# FASCICULE DE BREVET EUROPEEN

(11) **EP 0 810 903 B1**
(45) Date de publication et mention de la délivrance du brevet: **26.05.1999**
(21) Numéro de dépôt: 96937439.6
(22) Date de dépôt: 20.11.1996
(51) Int. Cl.: B01J 31/24, C07C 69/716

(54) **CATALYSEURS DE RUTHENIUM ET LEUR UTILISATION DANS L'HYDROGENATION ASYMETRIQUE DE CYCLOPENTENONES**
RUTHENIUMKATALYSATOREN UND DEREN VERWENDUNG ZUR ASYMMETRISCHEN CYCLOPENTENONHYDRIERUNG
RUTHENIUM CATALYSTS AND USE THEREOF IN ASYMMETRICAL CYCLOPENTENONE HYDROGENATION

(30) Priorité: 22.11.1995 CH 3300/95; 28.06.1996 CH 1621/96
(43) Date de publication de la demande: 10.12.1997
(73) Titulaire: FIRMENICH SA, 1211 Genève 8 (CH)
(72) Inventeur: RAUTENSTRAUCH, Valentin, CH-1233 Bernex (CH); VANHESSCHE, Koenraad, P., M., Union City, NJ 07087 (US); GENET, Jean-Pierre, F-91370 Verrières-le-Buisson (FR); LENOIR, Jean-Yves, F-76000 Rouen (FR)
(74) Mandataire: Salvaterra-Garcia, Maria de Lurdes
(86) Numéro de dépôt international: IB9601263
(87) Numéro de publication internationale: WO9718894

(56) Documents cités:
- EP-A- 0 470 756
- WO-A-91/02588
- WO-A-96/00206
- FR-A- 2 693 190
- NL-A- 6 918 228
- US-A- 5 304 524

## Description

### Domaine technique

La présente invention a trait au domaine de l'hydrogénation asymétrique en milieu homogène et, plus particulièrement, à l'utilisation de catalyseurs chiraux de Ru(II) nouveaux dans l'hydrogénation asymétrique de dérivés de la cyclopenténone obéissant à la formule générale 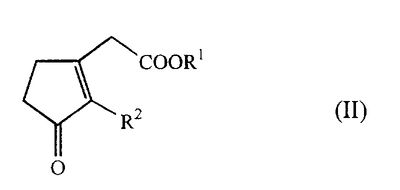 dans laquelle R¹ représente un radical alkyle linéaire ou ramifié de C₁ à C₄ et R² représente un reste d'hydrocarbure saturé ou insaturé, linéaire ou ramifié, de C₁ à C₈.

Les composés de formule (II) sont des substrats dont l'hydrogénation stéréospécifique et asymétrique s'était révélée jusqu'ici impraticable, en raison de leur faible potentiel donneur et de l'encombrement de leur structure, ceci malgré les progrès faits dans la catalyse homogène ces dernières années.

### Technique antérieure

On connait en effet, actuellement, de nombreux catalyseurs chiraux utiles dans l'hydrogénation asymétrique. Dans le contexte de la présente invention, il convient de citer en particulier les efforts de deux groupes de recherche qui se sont attelés à la synthèse de catalyseurs chiraux de Ru(II), obtenus à partir du complexe de Ru(II) de formule [(COD)Ru(2-méthylallyl)₂] (COD = cyclo-1,5-octadiène).

Ainsi, J.-P. Genet et ses collaborateurs ont publié des travaux ayant trait à des catalyseurs de formule [Ru(P*-P*)(2-méthylallyl)₂], dans laquelle P*-P* représente un ligand bidenté du type de ceux couramment connus sous des abréviations telles que DIOP, CHIRAPHOS, PROPHOS, BDPP, CBD, NORPHOS, DEGUPHOS, BPPM, BINAP, R-DuPHOS (R = méthyl ou éthyle), BIPHEMP ou encore DIPAMP (voir, par exemple, J.-P. Genet et al., Tetrahedron : Asymmetry 1991, 2, 43). Ces catalyseurs sont obtenus en chauffant le complexe de Ru(II) susmentionné avec le ligand diphosphiné approprié, dans un solvant tel que l'hexane ou le toluène, de façon à remplacer le cyclooctadiène par la phosphine chirale.

Lors de travaux subséquents (voir, par exemple, WO 91/02588; J.-P. Genet, Acros Organics Acta, 1994, 1, 1-8 ; J.-P. Genet et al., Tetrahedron : Asymmetry, 1994, 5, 665-690), ces auteurs ont décrit la transformation de ces catalyseurs par protonation à l'aide d'acides aqueux tels que HBr, HCl, HF ou HBF₄, dans des solvants fortement complexants, capables de jouer un rôle dans la stabilisation de la structure de coordination autour du métal, laquelle, selon ces auteurs, est de type hexacoordonné. Ce type de catalyseurs, qui peuvent être préparés in situ, s'est révélé utile dans l'hydrogénation asymétrique, dans des solvants protiques ou fortement électrodonneurs (méthanol, éthanol, THF, ou leurs mélanges avec d'autres solvants), de substrats possédant des groupes carbonyles et des liaisons éthyléniques acycliques.

D'autres travaux (voir, par exemple, F. Heiser et al., Tetrahedron : Asymmetry, 1991, 2, 51-62; EP 643 052; EP 398 132; EP 570 674) ont fait état de l'utilisation pour l'hydrogénation d'une variété de substrats, de catalyseurs préparés in situ à partir du même complexe de ruthénium, mais par un procédé selon lequel on traite un mélange de ce complexe et d'un ligand diphosphiné approprié avec notamment du CF₃COOH, de nouveau dans un solvant électrodonneur pouvant stabiliser la structure coordinative du métal.

Ces catalyseurs, et d'autres encore obtenus par des procédés similaires décrits dans les références citées, se révèlent très efficaces dans l'hydrogénation asymétrique de substrats variés, souvent des substrats bons donneurs pouvant complexer le Ru(II), et sont typiquement utilisés avec des solvants protiques, ou des mélanges de solvants protiques et non-protiques. Ils se sont cependant révélés inefficaces vis-à-vis des substrats de formule (II) cités plus haut, lorsque préparés selon les procédés de l'art antérieur et utilisés dans les conditions connues. Ces substrats (II) sont en effet de très faibles électrodonneurs, par rapport au type de substrats décrits dans l'art antérieur, et les nombreux efforts entrepris jusqu'ici pour leur hydrogénation asymétrique s'étaient toujours révélés infructueux.

Il convient de noter que, parmi les substrats de formule (II), le 3-oxo-2-pentyl-1-cyclopentène-1-acétate de méthyle présente un intérêt particulier dans le contexte de l'invention, dans la mesure où son hydrogénation asymétrique pourrait fournir, en une seule étape, des isomères optiquement actifs du dihydrojasmonate de méthyle ou Hédione® (origine : Firmenich SA, Genève, Suisse), un ingrédient parfumant très apprécié.

Parmi les quatre stéréoisomères de l'Hédione®, il est connu que le (+)-(1R)-cis-3-oxo-2-pentyl-1-cyclopentaneacétate de méthyle possède au mieux les caractères odorants, et notamment la note jasminée, recherchés dans l'Hédione®, la puissance de son odeur étant également de plusieurs ordres de grandeur supérieure à celle des autres isomères. Ainsi, l'obtention du (+)-(1R)-cis-3-oxo-2-pentyl-1-cyclopentaneacétate de méthyle optiquement pur, ou de mélanges d'isomères contenant essentiellement ce composé, revêt une importance capitale en parfumerie. Or, il n'existe à ce jour aucune synthèse de ce composé pouvant être exploitée à l'échelle industrielle et à partir d'un substrat tel que cité plus haut, tous les efforts d'hydrogénation asymétrique de ce substrat ayant échoué jusqu'à maintenant.

### Exposé de l'invention

La présente invention apporte justement une solution à ce problème.

Nous avons maintenant découvert que l'on peut obtenir cet isomère préféré de l'Hédione® avec une pureté diastéréochimique et énantiomérique excellente, à l'aide d'un procédé à une étape, faisant usage de catalyseurs chiraux de ruthénium nouveaux, obtenus selon un procédé original.

Un premier objet de l'invention est ainsi un catalyseur de ruthénium (II) comprenant des ligands constitués par des phosphines bidentées, caractérisé en ce qu'il est susceptible d'être obtenu par un procédé qui comprend le traitement d'un complexe de Ru (II) approprié et d'un ligand diphosphiné bidenté, présents en quantités équimolaires, avec un acide de formule HX, où X représente un anion non coordinant, cet acide étant utilisé dans une proportion ne dépassant pas 2 équivalents molaires par mole du complexe de Ru (II), le traitement ayant lieu dans un milieu non complexant ou faiblement complexant et sous atmosphère inerte.

Par "complexe de Pu (II) approprié", on entend ici tout complexe de ruthénium (II) dans lequel le métal est entouré de ligands de type diényle et allyle, de façon que le métal soit σ-lié à deux de ces ligands, lesquels possèdent en plus au moins une liaison π-liée au métal, deux autres positions de coordination du métal étant π-liées à ces deux mêmes ligands ou à un ligand distinct.

On connaît de l'art antérieur plusieurs composés de ruthénium, ou des composés analogues avec d'autres métaux de transition, comportant des ligands qui remplissent les conditions énoncées ci-dessus et conviennent ainsi en tant que précurseurs des catalyseurs de l'invention.

On peut citer plus particulièrement, en tant que complexes de ruthénium (II) appropriés, les composés de type [(diène) Ru (allyle) ₂], dans lesquels "diène" représente par exemple le COD (cycloocta-1,5-diène) ou le NBD (norbornadiène), ou encore le hepta-1,4-diène, et "allyle" représente un radical 2-propényle ou 2-méthallyle (voir, par exemple, J.-P. Genet et al., réfs citées ; M.O. Albers et al., Inorganic Synth. 1989, 26, 249 ; R.R. Schrock et al., J. Chem. Soc. Dalton Trans, 1974, 951). D'autres complexes de ruthénium (II) appropriés incluent les composés de type [bis (pentadiényl) Ru] dans lesquels "pentadiényl" représente un radical 2,4-diméthylpentadiényle, 2,3,4-triméthylpentadiényle, 2,4-di(tert-butyl)pentadiényle ou encore 2,4-diméthyl-1-oxapentadiényle (voir, par exemple. R.D. Ernst et al., J. Organometallic Chem., 1991, 402, 17 ; L. Stahl et al., Organometallic 1983, 2, 1229; T. Schmidt et al., J. Chem. Soc. Chem. Commun., 1991, 1427 ; T.D. Newbound et al., Organometallics, 1990, 9, 2962).

Selon un mode d'exécution préférentiel des catalyseurs de l'invention, on utilise en tant que précurseur de Ru (II) le composé de formule [(COD) Ru (2-méthallyle)₂], le bis(2,4-diméthylpentadiényl)ruthénium (voir L. Stahl et al. ou T.D. Newbound et al., réfs citées) ou les complexes bis(2,4-diméthyl-1-oxapentadiényl)ruthénium (voir T. Schmidt et al., réf. citée).

Parmi les phosphines bidentées pouvant être présentes en tant que ligands dans les catalyseurs de l'invention, on peut citer à titre préférentiel celles sélectionnées dans le groupe constitué par les phosphines chirales connues sous les abréviations de Me-DuPHOS, Et-DuPHOS, BINAP, TolBINAP, SKEWPHOS, DIPAMP et CHIRAPHOS, dont les structures sont représentées ci-après pour l'un des énantiomères en particulier.

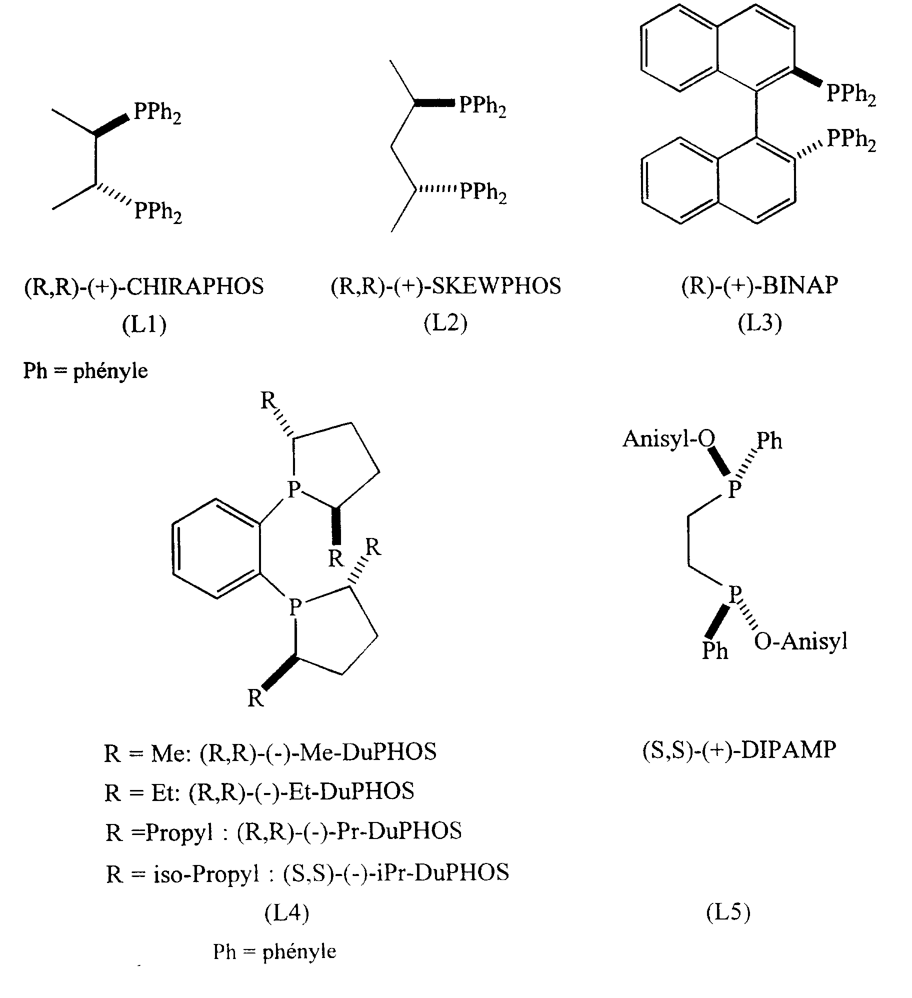

D'autres phosphines bidentées chirales pouvant être utilisées dans les catalyseurs chiraux de l'invention incluent par exemple celles connues sous le nom de NORPHOS, ou encore des analogues des ligands de type DuPHOS, dits "BPE", dont les structures sont représentées ci-après, pour l'un des énantiomères.

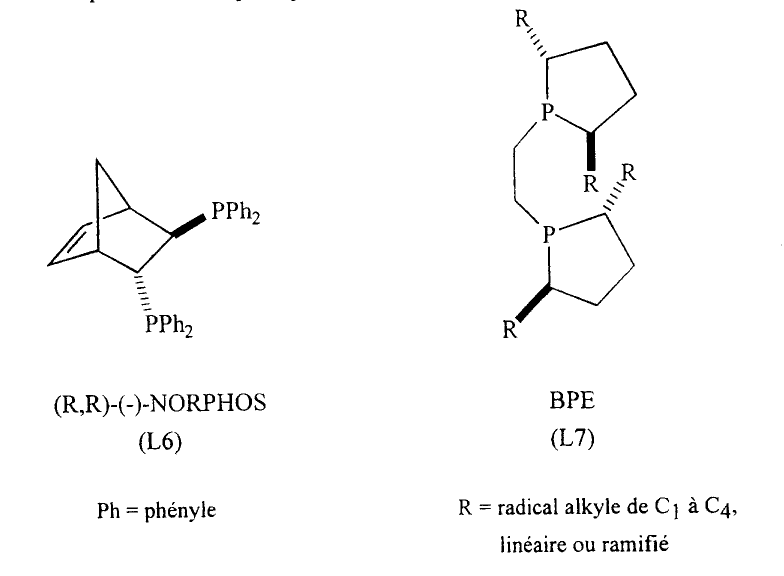

D'autres ligands particulièrement utiles pour la préparation des catalyseurs de l'invention sont les diphosphines chirales de formule 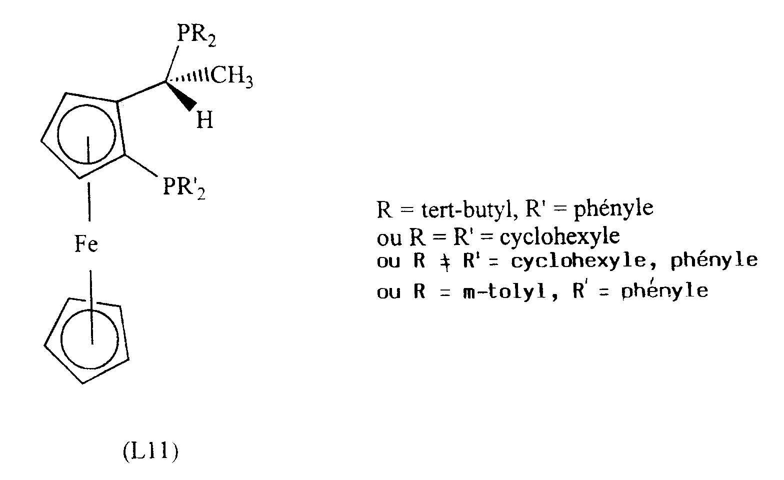 en particulier celle connue sous le nom de (R)-(S)-JOSIPHOS (R=cyclohexyle, R'=phényle) ou (-)-JOSIPHOS.

Nous avons, par ailleurs, constaté que d'autres ligands, chiraux ou achiraux, servaient à préparer des catalyseurs selon l'invention, lesquels se révélaient capables de catalyser l'hydrogénation des substrats (II) avec une stéréosélectivité cis supérieure à 80% et dans la plupart des cas, à 95%. A cet effet, on peut citer les ferrocényldiphosphines bidentées racémiques ou achirales telles que représentées ci-après.

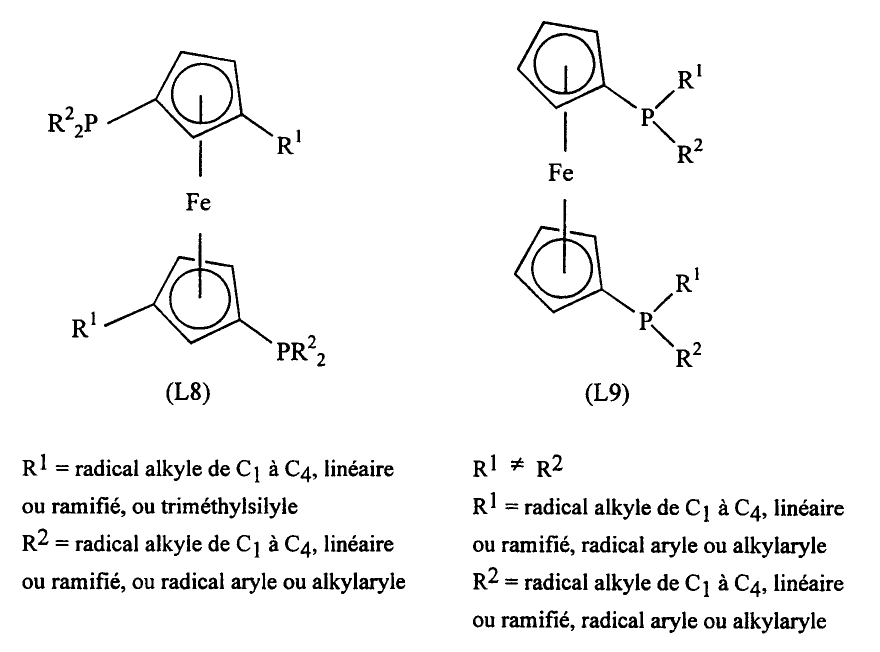

D'autres phosphines bidentées utiles en tant que ligands sont représentées ci-dessous :

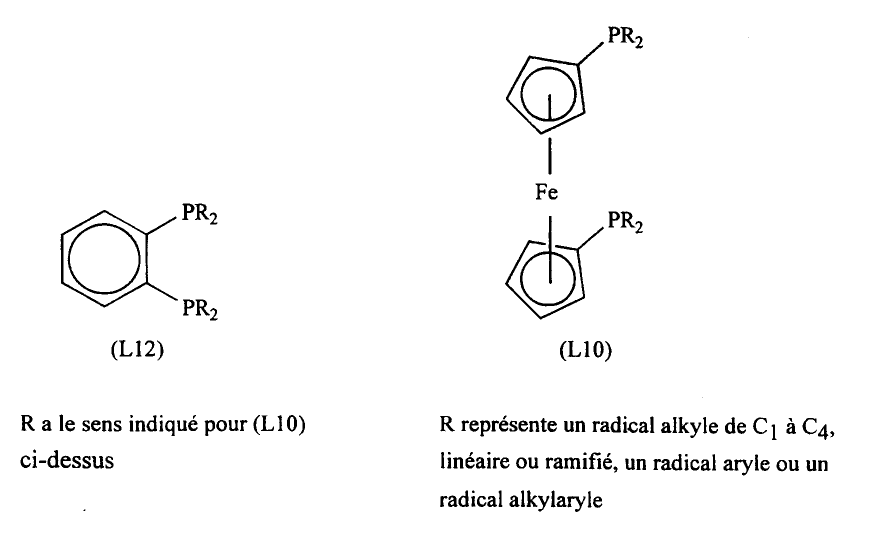

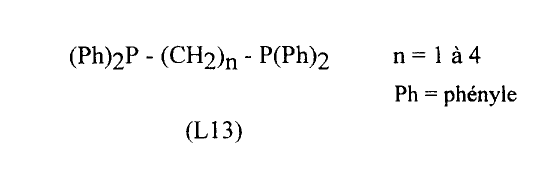

D'une façon générale, on peut utiliser en tant que ligand dans les catalyseurs de l'invention une diphosphine bidentée comportant des groupes substituants susceptibles de la rendre suffisamment riche en électrons pour lui permettre de stabiliser le métal, sans pour autant ôter à ce dernier la capacité de coordiner le substrat (II).

Nous avons ainsi constaté que les diphosphines possédant un certain nombre de substituants de type alkyle ou cycloalkyle se révélaient particulièrement utiles pour les buts de l'invention et fournissaient des catalyseurs très actifs et efficaces pour l'hydrogénation des substrats (II).

Il apparait de ce qui précède que les ligands préférés pour préparer les catalyseurs selon l'invention sont des ligands diphosphinés, dans lesquels les deux atomes de phosphore sont pontés par des groupes de type alkyle, 1,2-benzényle, bis(naphtalényle) ou encore 1,1'-ferrocényle, éventuellement substitués, lesdits atomes de phosphore portant par ailleurs deux autres substituants, qui peuvent être identiques ou distincts et constitués par des radicaux alkyles, aryles ou alkylaryles, ou encore alicycliques.

Les catalyseurs de l'invention comprenant des ligands diphosphinés de type DuPHOS, BINAP, TolBINAP, SKEWPHOS ou JOSIPHOS se sont révélés particulièrement avantageux en tant que catalyseurs d'hydrogénation asymétrique des substrats de formule (II), comme il ressort des exemples présentés plus loin.

Parmi ces catalyseurs, ceux comprenant les ligands de type SKEWPHOS, JOSIPHOS ou Me-DuPHOS se sont montrés capables de performances particulièrement avantageuses et sont donc préférés selon l'invention. Le ligand (R,R)-(-)-Me-DuPHOS ou (-)-1,2-bis(2,5-diméthylphospholano)benzényle a permis d'obtenir des catalyseurs de choix selon l'invention.

Les ligands de type L1 a L13 représentés ci-dessus sont des composés pouvant être obtenus commercialement ou préparés selon des procédés analogues à des procédés décrits auparavant.

Par exemple, les ligands de type DuPHOS, SKEWPHOS, BINAP, CHIRAPHOS, DIPAMP et NORPHOS sont, pour la plupart, des produits commerciaux et, en tout cas, on peut les obtenir par des méthodes décrites dans la littérature, notamment dans des oeuvres de référence telles que les livres de R. Noyori, Asymmetric Catalysis in Organic Synthesis, John Wiley & Sons N.Y (1994), Chap. II et J. Ojima, Catalytic Asymmetric Synthesis, VCH Publishers, N.Y. (1994), Chap. I.

Les ligands de type L13 sont très courants et commercialisés, alors que ceux contenant des groupes ferrocényle sont soit commercialisés (c'est le cas par exemple du composé de structure L10 dans laquelle R représente un radical phényle), soit ils peuvent être préparés par des méthodes analogues à celles décrites par exemple par M. D. Rausch et al., J. Organometallic Chem. 1967, 10, 127, R. A. Brown et al. 1992, 20, 2611 et G. Herberich et al., Chem. Ber. 1995, 128, 689. Certains de ces ligands de type ferrocényldiphosphine sont par ailleurs des composés nouveaux qui font également l'objet de l'invention. C'est le cas notamment des ligands de formule (L8) dans lesquels R¹=triméthylsilyle et R²=isopropyle.

Les ligands de type (L11) dans lesquels R=cyclohexyle et R'=phényle, connus sous la désignation de JOSIPHOS, peuvent être préparés comme il est décrit par A. Togni et al., J. Amer. Chem. Soc. 1994, 116, 4062. Ils sont également disponibles commercialement, ainsi que leurs analogues (origine : STREM Chemicals, Inc.).

Les acides de formule HX mentionnés plus haut, servant à préparer les catalyseurs de l'invention, sont typiquement utilisés sous forme des éthérates correspondants (par exemple HBF₄.R₂O, R=CH₃ ou C₂H₅) ou de tout autre sel de type onium (phosphonium ou sulfonium, par exemple). De tels éthérates sont disponibles commercialement ou préparés à partir des sels d'argent correspondants, par réaction avec HCl. Dans ce dernier cas, on fera typiquement réagir le sel d'argent, par exemple AgBF₄, AgPF₆, AgSbF₆ ou AgAsF₆, avec HCl, dans un solvant contenant un dialkyléther, par exemple un mélange de dichlorométhane et éther diéthylique. Le chlorure d'argent précipite, fournissant ainsi la solution de l'éthérate de l'acide, qui est ensuite utilisée selon l'invention dans la réaction avec le complexe de ruthénium et le ligand phosphiné.

On peut citer, ainsi, à titre d'acide HX, un acide sélectionné dans le groupe constitué par HBF₄, HPF₆, HSbF₆, HAsF₆, et HB[3,5-(CF₃)₂C₆H₄]₄. Tous ces acides ont comme caractéristique commune le fait que leur anion est non coordinant.

Selon un mode d'exécution préféré du catalyseur de l'invention, on utilise l'éthérate de l'acide tétrafluoroborique.

La réaction qui caractérise le procédé d'obtention des catalyseurs de l'invention a lieu dans un milieu non complexant ou faiblement complexant, sous atmosphère inerte. On veut dire par cela, une atmosphère inerte dont le contenu en oxygène est inférieur à 200 ppm et, de préférence, ne dépasse pas 5 à 10 ppm.

Par milieu non complexant ou faiblement complexant on veut dire, par exemple, un solvant non complexant ou faiblement complexant. On peut vouloir dire par ailleurs que la réaction a lieu dans un solvant tel que défini ci-dessus, mais aussi en présence du substrat cité plus haut, de formule 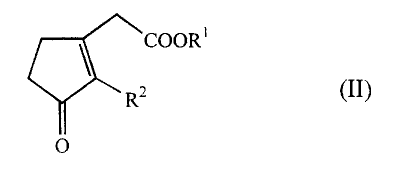 dans laquelle R¹ représente un radical alkyle linéaire ou ramifié de C₁ à C₄ et R² représente un reste d'hydrocarbure saturé ou insaturé, linéaire ou ramifié, de C₁ à C₈.

Nous avons encore constaté que des catalyseurs utiles étaient aussi obtenus en l'absence de solvant, et en présence uniquement du substrat de formule (II). C'est de façon surprenante que nous avons observé que la présence de ce substrat permettait aussi la formation d'espèces dicationiques de Ru(II) capables elles-mêmes de catalyser l'hydrogénation desdits substrats (II), dans les conditions des procédés qui font l'objet de l'invention, et qui sont décrits en détail ci-après.

Lorsque le traitement du complexe de Ru(II) et du ligand diphosphiné, éventuellement chiral, avec l'acide HX a lieu dans un solvant, celui-ci sera choisi de préférence dans le groupe constitué par le dichlorométhane, le dichloroéthane, le pivalate de méthyle, l'acétate de méthyle, l'acétate d'éthyle, l'acétate d'isopropyle, l'acétone, la 2-butanone, la 3-pentanone et tout mélange de deux ou plusieurs de ces solvants.

Selon un mode d'exécution préférentiel, on utilise le dichlorométhane ou des mélanges de celui-ci avec d'autres solvants non-complexants ou faiblement complexants, notamment ceux cités ci-dessus.

Il convient de noter que l'utilisation des solvants complexants typiquement employés pour préparer les catalyseurs de l'art antérieur n'a pas permis d'obtenir des catalyseurs capables de fournir, lors de leur utilisation dans l'hydrogénation du substrat (II) approprié, essentiellement l'isomère préféré susmentionné de l'Hédione®, ce qui ressort clairement des exemples présentés plus loin. Ceci, contrairement à ce que nous avons pu observer avec les catalyseurs selon la présente invention.

Or, la structure des catalyseurs dicationiques de Ru(II) de l'invention n'est pas connue. Sans vouloir préjuger de la nature exacte de leur structure, il paraît cependant probable qu'ils obéissent à une formule du type [(diène)Ru(P*-P*)S₂]²⁺(anion⁻)₂, dans laquelle le ligand S représente le solvant (par exemple CH₂Cl₂ et/ou éther), le substrat, ou un mélange des deux, et P*-P* représente la phosphine bidentée, éventuellement chirale. En effet, étant donné le faible caractère électrodonneur du solvant et/ou du substrat (II), et au vu des connaissances de l'art antérieur, il est tout à fait surprenant d'observer la formation, et une activité d'hydrogénation quelconque, de ces catalyseurs, alors que la structure coordinative autour du métal ne peut pas être totalement définie sur la base des connaissances dans l'art, par analogie à celle des catalyseurs préparés selon les méthodes connues.

Ces catalyseurs se sont révélés d'une efficacité excellente, là où les catalyseurs obtenus selon les procédés de l'art antérieur ont échoué. Nous avons en effet établi que leur utilisation pour l'hydrogénation, le cas échéant asymétrique, des composés (II) permettait d'obtenir des isomères de composés de formule 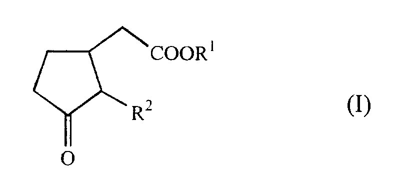 dans laquelle R¹ et R² ont le sens indiqué plus haut, essentiellement sous une configuration cyclanique cis et, le cas échéant, de façon énantiosélective, en fonction de la chiralité du catalyseur et avec une pureté optique excellente.

Plus particulièrement, nous avons pu obtenir le (+)-(1R)-cis-3-oxo-2-pentyl-1-cyclopentaneacétate de méthyle avec une pureté cis supérieure à 98% et un excès énantiomérique d'au moins 60%.

Parmi ces catalyseurs, et comme il ressort des exemples présentés plus loin, ceux obtenus à partir du complexe de formule [(COD)Ru(2-méthallyl)₂] et du ligand (-)-Me-DuPHOS ou (-)-JOSIPHOS, en utilisant l'éthérate de HBF₄, dans un milieu constitué par du dichlorométhane, ou des mélanges de celui-ci avec le 3-oxo-2-pentyl-1-cyclopentène-1-acétate de méthyle, se sont révélés particulièrement avantageux pour la préparation de la (+)-cis-Hédione®.

Les catalyseurs contenant les ligands chiraux cités auparavant se sont révélés particulièrement utiles dans la préparation de composés optiquement actifs de formule (I) et notamment du (+)-(1R)-cis-3-oxo-2-pentyl-1-cyclopentaneacétate de méthyle.

Bien entendu, l'utilisation des énantiomères des ligands cités ci-dessus permet d'obtenir l'énantiomère de configuration (-)-(1S) du cis-3-oxo-2-pentyl-1-cyclopentaneacétate de méthyle, puisque la configuration optique du catalyseur conditionne celle du produit de l'hydrogénation du substrat (II).

Selon l'invention, la préparation du catalyseur peut avoir lieu à température ambiante ou à une température inférieure. La température du traitement susmentionné ne semble d'ailleurs pas être un paramètre critique du procédé, susceptible d'influencer les propriétés du catalyseur et son efficacité dans l'hydrogénation des substrats (II). L'utilisation de la température ambiante se révèle, bien entendu avantageuse d'un point de vue pratique.

Par contre, nous avons observé que le rapport pondéral molaire de l'acide HX, par exemple, HBF₄, HPF₆, HSbF₆ ou HAsF₆, relativement au poids molaire de complexe de ruthénium, était un paramètre critique, conditionnant les caractéristiques du catalyseur et son efficacité dans l'hydrogénation asymétrique selon l'invention des substrats susmentionnés, et plus particulièrement du 3-oxo-2-pentyl-1-cyclopentène-1-acétate de méthyle. Ceci est d'autant plus surprenant que l'art antérieur est totalement muet à ce sujet. En effet, les études connues rapportent souvent l'utilisation d'un excès substantiel d'acide, par rapport à la quantité requise pour former le complexe cationique de ruthénium en présence du ligand phosphiné bidenté.

Or, nous avons constaté que l'utilisation d'un excès d'acide rend les catalyseurs inactifs ou inefficaces dans le contexte de la présente invention. Comme il ressort des exemples présentés plus loin, il peut se révéler même avantageux d'utiliser l'acide dans une proportion inférieure à la quantité stoichiométrique. Ainsi on utilisera cet acide, de préférence, dans une proportion comprise entre 1,5 et 2 équivalents molaires, par mole de complexe de Ru(II). Plus préférentiellement, le rapport entre ces deux réactifs sera d'environ 2 équivalents molaires d'acide par mole de complexe.

De même, le rapport équimolaire du complexe de Ru(II) approprié et de la diphosphine bidentée semble aussi être un paramètre critique de l'invention.

L'invention concerne également le procédé de préparation des catalyseurs de Ru(II), caractérisé en ce qu'on fait réagir, sous atmosphère inerte et dans un milieu non complexant ou faiblement complexant, des quantités équimolaires d'un complexe de Ru(II) approprié et d'un ligand diphosphiné bidenté, avec un acide de formule HX, où X représente un anion non coordinant, cet acide étant utilisé dans une proportion ne dépassant pas 2 équivalents molaires par mole de complexe de Ru(II).

Comme il a été mentionné auparavant, les complexes de Ru(II) appropriés, et notamment le complexe préféré [(COD)Ru(2-méthylallyl)₂], utilisés comme produits de départ dans le procédé d'obtention des catalyseurs de l'invention, sont typiquement des composés connus qui peuvent être préparés comme il est décrit dans les documents de l'art antérieur cités plus haut, ou selon des procédés analogues à ceux connus.

D'autre part, les phosphines chirales constituant les ligands des catalyseurs de l'invention, et déterminant leur chiralité, sont des composés commercialement disponibles ou qui peuvent être préparés par des procédés connus.

Comme cité plus haut, la préparation du catalyseur peut avoir lieu dans un milieu constitué par un solvant ou des mélanges du solvant avec le substrat (II). Ce dernier mode d'exécution, de même que celui selon lequel le catalyseur est préparé en présence uniquement du substrat (II), sont des méthodes particulièrement adaptées à la préparation in situ des catalyseurs, dans le milieu de la réaction d'hydrogénation du substrat, décrite plus loin.

Les catalyseurs de l'invention ainsi préparés sont obtenus sous forme de solutions dans le solvant et/ou le substrat (II), du produit résultant de la réaction du complexe de Ru(II) avec les ligands diphosphinés et l'acide HX. Ces solutions catalytiques peuvent être utilisées telles quelles pour l'hydrogénation asymétrique des substrats (II). Elles peuvent être conservées sous atmosphère inerte et restent actives pendant plusieurs jours. Par ailleurs, elles peuvent également être concentrées sous vide pour fournir les catalyseurs sous forme solide.

Les catalyseurs peuvent également être engendrés in situ dans le milieu d'hydrogénation, comme il ressort de ce qui suit.

Dans ce procédé, l'ordre de mélange des réactifs ne semble pas avoir une influence critique sur les propriétés des catalyseurs qui en résultent. Ainsi, nous avons constaté que l'on pouvait mélanger d'abord le précurseur de Ru(II), par exemple le [(COD)Ru(2-méthallyl)₂] avec le ligand diphosphiné, notamment le (-)-Me-DuPHOS, dans le solvant, typiquement le dichlorométhane, en ajoutant ensuite à ce mélange l'acide choisi, par exemple du HBF₄ (sous forme de son éthérate), éventuellement en solution dans le dichlorométhane. Des solutions catalytiques selon l'invention sont ainsi obtenues, ayant une concentration variable en catalyseur selon l'invention, par exemple de l'ordre de 0,01 M (0,01 moles de catalyseur/l de solution catalytique), qui se sont révélées très avantageuses dans l'hydrogénation des substrats (II).

Alternativement, de très bons catalyseurs ont également été obtenus en mélangeant d'abord le ligand diphosphiné, notamment le (-)-Me-DuPHOS, avec l'acide de formule HX, sous forme d'éthérate ou d'un sel onium, en particulier le HBF₄.éthérate, dans le dichlorométhane par exemple. Le produit ainsi obtenu peut être réduit à la forme d'un sel onium solide, en enlevant l'éther et le CH₂Cl₂ sous vide, et ensuite utilisé dans la réaction avec le complexe de Ru(II), dans un solvant, pour fournir les catalyseurs désirés. Ce mode d'exécution est très utile en pratique car il permet la préparation séparée des sels du ligand, lesquels peuvent agir ensuite sur le complexe de Ru(II).

Les composés dicationiques de Ru(II) de l'invention se révèlent très utiles en tant que catalyseurs de réactions d'hydrogénation. En particulier, ils sont d'une efficacité excellente dans la conversion des substrats (II) et l'invention concerne donc également un procédé pour la préparation d'un composé de formule 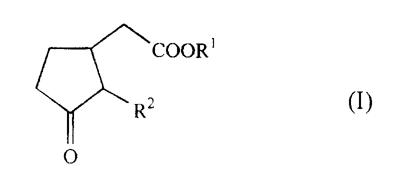 dans laquelle R¹ représente un radical alkyle linéaire ou ramifié de C₁ à C₄ et R² représente un reste d'hydrocarbure saturé ou insaturé, linéaire ou ramifié, de C₁ à C₈, essentiellement sous forme d'un isomère de configuration cyclanique cis, caractérisé en ce qu'on soumet à une hydrogénation catalytique un substrat de formule 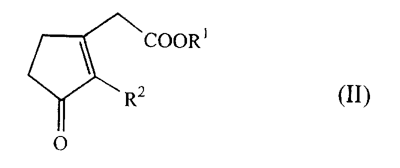 dans laquelle R¹ et R² ont le sens indiqué ci-dessus, en présence d'un catalyseur de Ru(II) selon l'invention, tel que décrit auparavant, et à une pression d'hydrogène comprise entre 10 et 100 bar (10 et 10x10⁶ Pa).

Selon un mode d'exécution préféré du procédé de l'invention, le catalyseur est engendré in situ avant l'hydrogénation du substrat (II) ou éventuellement en présence de celui-ci, par traitement de quantités équimolaires du complexe de Ru(II), notamment le composé de formule [(COD)Ru(2-méthylallyl)₂] et d'un ligand chiral phosphiné bidenté approprié, sélectionné par exemple dans le groupe constitué par des diphosphines connues sous les abréviations de Me-DuPHOS, Et-DuPHOS, BINAP, TolBINAP, SKEWPHOS, DIPAMP, CHIRAPHOS et JOSIPHOS, avec l'éthérate d'acide tétrafluoroborique dans une proportion d'environ 2 équivalents molaires d'HBF₄ par mode du complexe de Ru(II). Ce mode d'exécution préféré permet d'obtenir le composé (I) susmentionné sous forme d'un isomère optiquement actif de configuration cyclanique (1R)-cis.

Tous les ligands racémiques ou achiraux mentionnés plus haut fournissent des catalyseurs permettant d'obtenir les composés (I) sous forme de l'isomère de configuration cis racémique. Bien entendu, dans le cas des ligands racémiques (par exemple L8 et L9), on peut obtenir les énantiomères correspondants par séparation du ligand racémique, et ces énantiomères du ligand peuvent ensuite servir à préparer des catalyseurs optiquement actifs selon l'invention.

Lorsque la préparation du catalyseur a lieu en absence du substrat (II), on préparera de préférence d'abord le mélange de complexe de Ru(II) et de ligand diphosphiné sous forme de solution dans un solvant non-complexant ou faiblement complexant et on ajoutera à cette solution l'acide comportant l'anion non-coordinant, notamment l'éthérate d'acide tétrafluoroborique, généralement en solution dans le même solvant, ou dans un autre solvant ayant les mêmes caractéristiques. La solution catalytique ainsi obtenue sera alors utilisée pour hydrogéner le substrat (II).

Si l'on utilise un milieu d'hydrogénation comprenant un solvant on utilisera, de préférence, le même que celui dans lequel le catalyseur est obtenu. A cet effet on peut utiliser un hydrocarbure chloré tel que le dichlorométhane, le dichloroéthane ou des mélanges des deux. L'utilisation de dichlorométhane, éventuellement en mélange avec d'autres solvants tels que cités plus haut, s'est révélée très avantageuse selon l'invention.

Selon un mode d'exécution particulièrement avantageux du procédé d'hydrogénation de l'invention, on utilise en tant que substrat de formule (II) le 3-oxo-2-pentyl-1-cyclopentène-1-acétate de méthyle pour obtenir l'Hédione® sous forme de son isomère de configuration (+)-cis préféré.

Les catalyseurs de l'invention possédant des ligands de type DuPHOS, et en particulier Me-DuPHOS, se sont révélés particulièrement utiles dans l'hydrogénation de ce substrat.

L'hydrogénation qui caractérise le procédé de l'invention est effectuée sous une pression d'hydrogène pouvant varier entre 10 et 100 bar (2x10⁶ et 10x10⁶ Pa). Des pressions comprises entre 70 et 100 bar, et plus particulièrement entre 90 et 100 bar, sont préférées.

La réaction peut avoir lieu à des températures allant jusqu'à 50 à 60° C, ou même 100°C. De préférence, on utilisera la température ambiante ou une température inférieure. Il a été constaté que des températures allant jusqu'à -10°C, ou même inférieures, permettaient d'obtenir des résultats très utiles.

Les concentrations molaires du catalyseur de Ru(II) par rapport au substrat varient typiquement entre 0,01 et 4 mole % et, de préférence entre 0,1 et 2 mole %. Selon un mode d'exécution encore plus préférentiel, elles seront de l'ordre de 0,3 ou 0,4 mole %.

Par ailleurs, il a aussi été constaté qu'il convenait d'utiliser des solutions concentrées en substrat, de meilleurs résultats étant obtenus lorsque la concentration de substrat dans le milieu d'hydrogénation était comprise entre environ 0,4 et 1,5 Molaire, par rapport au volume dudit milieu.

On peut aussi ajouter à ce milieu une trialkylamine, de préférence la diisopropyléthylamine (DIPEA). Il a en effet été observé que, dans certaines conditions, les hydrogénations effectuées en présence de cette dernière donnaient des résultats plus utiles. La concentration dans laquelle cette base peut être ajoutée au milieu réactionnel varie dans un rapport molaire de 0,1 : 1 à 0,5 : 1, par rapport au catalyseur de Ru(II). De meilleurs rendements en produit final ont été obtenus lorsque l'on a ajouté la diisopropyléthylamine dans une proportion de 0,2 éq. par rapport au catalyseur.

Selon un mode d'exécution particulier du procédé d'hydrogénation de l'invention, dans lequel le catalyseur est engendré in situ dans le milieu d'hydrogénation, on mélange d'abord le substrat à hydrogéner avec le mélange équimolaire de [(COD)Ru(2-méthylallyl)₂] et de ligand diphosphiné, éventuellement dans un solvant, et on ajoute à la solution ainsi obtenue l'éthérate d'HBF₄, ou un autre sel d'acide HX, dans la proportion mentionnée plus haut. Le mélange réactionnel ainsi obtenu est ensuite mis sous pression d'hydrogène de façon conventionnelle, comme il est décrit dans les exemples présentés plus loin. Cet éthérate ou autre sel de type onium peut être ajouté tel quel ou sous forme de solution, soit dans le même solvant que celui utilisé auparavant, soit dans un solvant différent.

L'invention fournit ainsi des catalyseurs et des procédés d'utilisation de ces catalyseurs permettant d'obtenir, en une seule étape, dans des conditions avantageuses et susceptibles d'application industrielle, de produits utiles en parfumerie, de façon stéréosélective et, le cas échéant, énantiosélective et avec des rendements excellents. Les catalyseurs de l'invention, contrairement à ceux de l'art antérieur, sont capables d'hydrogéner les substrats (II) avec des vitesses de réaction très rapides, des taux de conversion du substrat supérieurs à 98% et en fournissant essentiellement le diastéréomère de configuration (1R)-cis des composés désires.

L'invention sera maintenant décrite plus en détail à l'aide des exemples suivants, dans lesquels les températures sont indiquées en degrés Celsius et les abréviations ont le sens courant dans l'art.

### Manières de réaliser l'invention

### Exemple 1

### Préparation des catalyseurs

### Méthode générale

Dans une boîte à gants, sous argon, à température ambiante, on a introduit dans un tube Schlenk des quantités équimolaires de [(COD)Ru(2-méthylallyl)₂], ou un autre complexe de Ru, et du ligand choisi. On a ajouté le solvant séché et dégazé nécessaire pour diluer ce mélange. Après 5 min, on a ajouté goutte à goutte, à l'aide d'une seringue, l'éthérate d'acide tétrafluoroborique (ou un autre sel onium), éventuellement en solution dans le solvant non-complexant, en agitant à l'aide d'un barreau magnétique couvert de Teflon®. Après agitation, on a ajouté alors encore un peu de solvant et agité pendant environ une demi-heure. Le catalyseur ainsi obtenu sous forme de solution peut être stocké pendant plusieurs jours dans la boîte à gants sans perte d'activité.
Cette solution peut aussi être concentrée sous vide pour fournir le catalyseur sous forme solide.

Dans un essai typique, on a fait réagir 0,20 mmole (64 mg) de [(COD)Ru(2-méthylallyl)₂] (origine : Acros Organics) et 0,20 mmole (61 mg) de (-)-(R,R)-Me-DuPHOS ou [(-)-1,2-bis-(2R,5R)-2,5-diméthylphospholano)-benzène ; origine : Strem Chemicals] dans 4 ml de CH₂Cl₂. Une solution (4 ml) à 0,10 N de HBF₄.Et₂O (Et = éthyle ; 0,40 mmole) dans le CH₂Cl₂ a été ajoutée. Après 30 min d'agitation, on a encore ajouté 8 ml de CH₂Cl₂ et agité une demi-heure. Le catalyseur ainsi obtenu a été utilisé tel quel dans les réactions d'hydrogénation.
Des catalyseurs optiquement actifs ont ainsi été préparés avec les ligands suivants : (+)-(S,S)-Me-DuPHOS ; (-)-(R,R)-Me-DuPHOS ; (+)-(S,S)-Et-DuPHOS ; (-)-(R,R)-Et-DuPHOS ; (+)-(R)-BINAP ; (-)-(S)-BINAP ; (-)-(R,R)-NORPHOS ; (-)-(S,S)-CHIRAPHOS; (+)-(R,R)-CHIRAPHOS ; (-)-(SS)-SKEWPHOS ; (+)-(R,R)-SKEWPHOS ; (-)-(R,R)-DIPAMP ; (+)-(S,S)-DIPAMP ; (-)-(S)-TolBINAP ; (+)-(R)-TolBINAP ; (-)-(R)-(S)-JOSIPHOS et (+)-(S)-(P)-JOSIPHOS. Tous ces ligands sont disponibles commercialement ou préparés par des méthodes connues.

D'autres catalyseurs ainsi préparés, selon la méthode décrite ci-dessus, comprenaient des ligands de type L7 (R=méthyle, éthyle ou isopropyle), L8 [R¹=(CH₃)₃Si et R²=phényle ou isopropyle], L9 (R¹=CH₃ et R²=phényle), L10 (R=éthyle, isopropyle ou phényle), L11 (R=cyclohexyle et R'=phényle), L12 (R=phényle) et L13 (n=1, 2, 3 ou 4).
Les ligands L13 susmentionnés sont disponibles sur le marché (origine : Fluka Chemie et Aldrich) et de même le ligand de type L12 cité (origine : Strem Chemicals).
Les ligands susmentionnés de type L8, L9 et L10 ont été préparés par des méthodes connues (voir références citées plus haut et I.R.Butter et al., Synth. React. Inorg. Met. - Org. Chem. 1985, 15, 109), à partir du ferrocène et selon les schémas que voici :

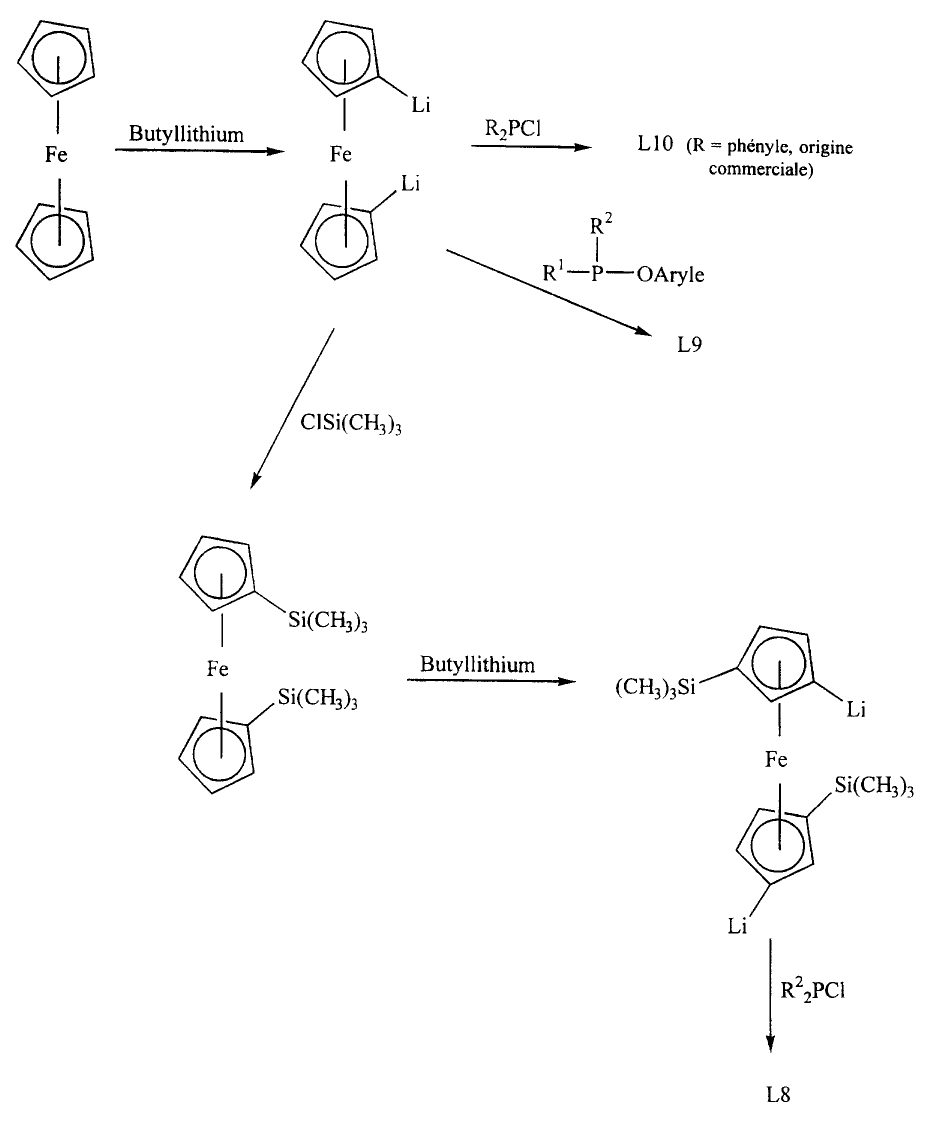

Pour la préparation des ligands de type L7, on peut utiliser la méthode décrite par exemple dans US 5,171,892.
Dans la méthode de préparation du catalyseur décrite plus haut, on a également utilisé une solution de HBF₄.Et₂O dans l'éther diéthylique (0,2 M), avec des résultats similaires.

### Exemple 2

### Hydrogénation du 3-oxo-2-pentyl-1-cyclopentène-1-acétate de méthyle

### Méthode générale d'hydrogénation

Dans une boîte à gants sous argon et à température ambiante, on a introduit, dans un gobelet en verte adapté à l'usage dans un autoclave, la solution du catalyseur obtenu comme il est décrit dans l'exemple 1, le substrat susmentionné, qui avait été dégazé au préalable et le solvant dégazé. Le gobelet contenant un barreau d'agitation a été introduit dans l'autoclave à l'intérieur de la boîte à gants et cet autoclave a été scellé avant d'être retiré de la boite à gants pour pressurisation. On a purgé les entrées de l'autoclave à l'aide de H₂ et ensuite pressurisé à la valeur choisie. On a laissé réagir à la température ambiante. On a dégazé à l'air, ouvert l'autoclave et concentré la solution sous pression réduite. On a précipité le catalyseur à l'aide de pentane, filtré et concentré le filtrat pour obtenir l'Hédione® désirée. Le produit peut être purifié par distillation au four à boules (150-200°C/5 Pa) sans altération du rapport diastéréomérique.

Par exemple, dans un essai typique, on a utilisé une pression de H₂ de 90 bar (9x10⁶ Pa), température ambiante et un temps de réaction de 1,5 h pour un poids de substrat de 269 mg (1,20 mmole) et 1 ml de CH₂Cl₂, le catalyseur étant présent à raison de 1 mole % par rapport au substrat (1,0 ml de solution du catalyseur de (-)-(R,R)-Me-DuPHOS décrit spécifiquement à l'exemple 1).

Le Tableau I ci-après résume les résultats de différents essais, effectués dans des conditions variées et avec les catalyseurs préparés comme il est décrit à l'exemple 1, à l'aide des ligands chiraux indiqués au Tableau.
Le remplacement des ligands mentionnés dans ce Tableau par leurs énantiomères respectifs a permis d'obtenir des produits où le rapport isomérique cis/trans était identique à la valeur correspondant à l'énantiomère en question indiquée au Tableau, mais où le rapport (+)-(cis)/(-)-cis et (-)-trans/(+)-trans était l'inverse de celui indiqué au Tableau. Une analyse de ce tableau montre que lors de l'utilisation de solvants complexants tels que le méthanol (essais 1 et 2) ou encore des mélanges de solvants complexants et non-complexants (essai 11), soit on a de mauvais rendements en Hédione®, soit on obtient des rapports cis/trans défavorables, ceci en comparaison avec les essais effectués par exemple dans le dichlorométhane. Par ailleurs lorsque le substrat est trop dilué (essai 7), on n'observe pas d'hydrogénation.

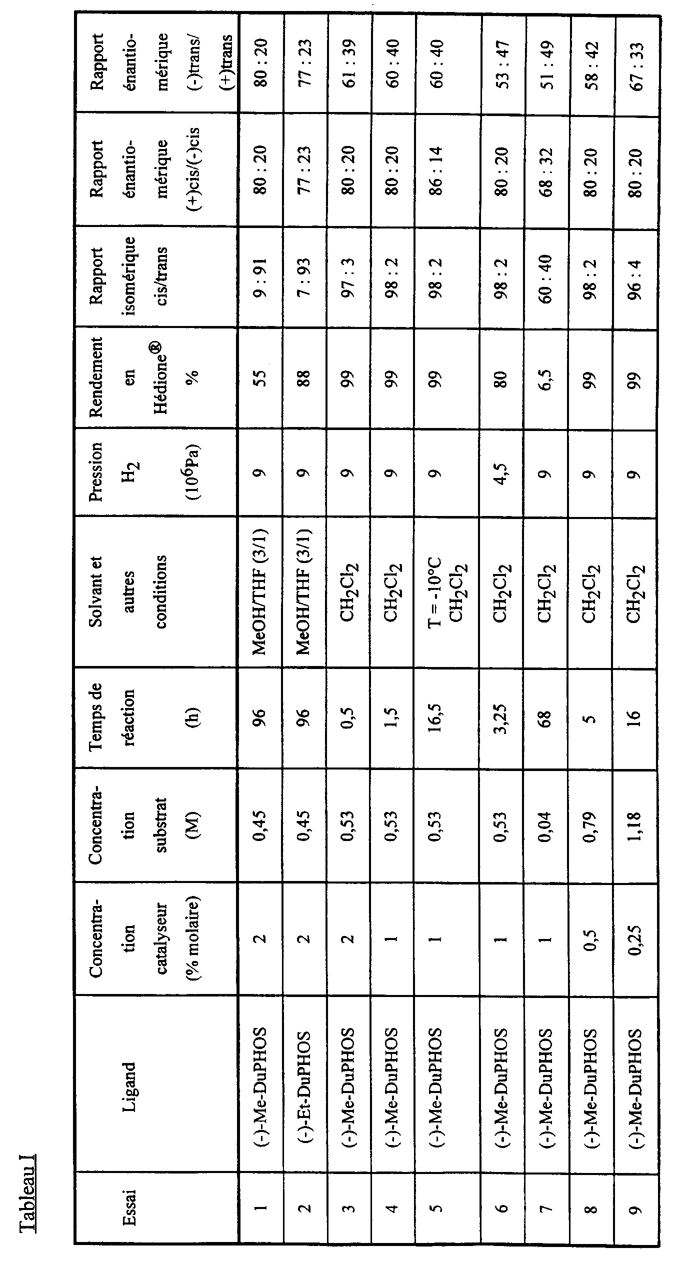

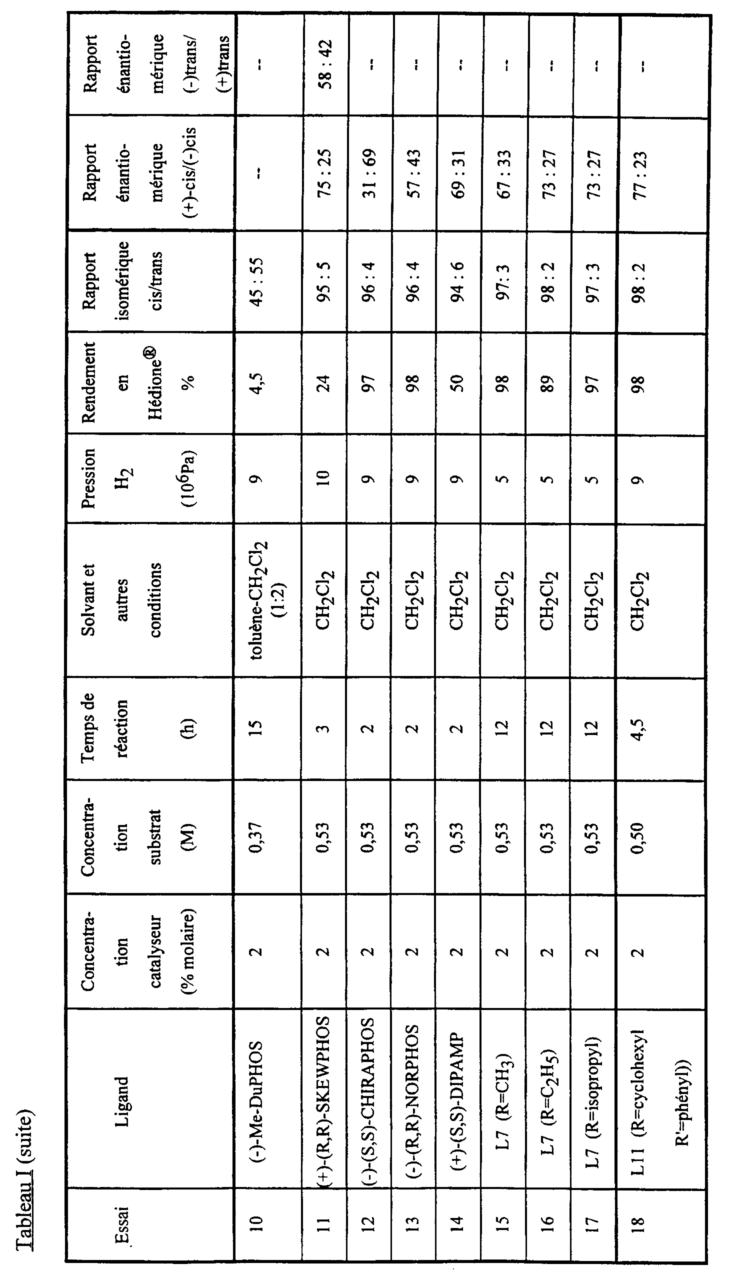

### Exemple 3

On a hydrogéné le substrat cité à l'exemple 2 selon une méthode analogue à celle décrite auparavant, en procédant ainsi.
Dans une boîte à gants sous Ar, à température ambiante, on a dissous le [(COD)Ru(2-méthylallyl)₂] (16,5 mg ; 0,051 mmole) dans du dichlorométhane (1 ml) et agité pendant 2 min. A la solution résultante on a ajouté une solution de (+)-R-BINAP [(+)-(R)-2,2'-bis(diphénylphosphino-1,1'-binaphtalène) ; 31,6 mg ; 0,051 mmole ; origine : Fluka Chemicals] dans du dichlorométhane (2 ml) et agité pendant environ 30 min. On a ensuite ajouté de l'éthérate d'acide tétrafluoroborique (14 µl ; origine : Fluka Chemie). Après 30 min d'agitation, on a ajouté une solution de 3-oxo-2-pentyl-1-cyclopentèneacétate de méthyle (0,5 g ; 2,23 mmole) dans du dichlorométhane (10 ml) et agité une demi-heure à température ambiante. Ce mélange, toujours sous Ar, a été chargé dans un autoclave contenant un gobelet en verte avec agitateur magnétique. Après avoir purgé avec H₂, on a pressurisé à 9x10⁶ Pa d'hydrogène pendant 64 h à 20°. On a décompressé et prélevé la solution orange qui a été concentrée sous vide poussé. Le résidu a été pris dans le pentane (2 ml) pour précipiter le catalyseur. On a filtré sur acrodisc 0,45 µm et concentré. On a ainsi obtenu 500 mg d'une huile transparente contenant 93% d'Hédione®, avec un rapport cis/trans de 72 : 28, et 6% de produit de départ. Les rapports énantiomériques étaient les suivants (+)-cis/(-)-cis = 73/27 ; (+)-(trans)/(-)-trans = 35/65.

### Exemple 4

Dans une boîte à gants sous argon et à température ambiante on a introduit, dans un gobelet en verte adapté à l'usage dans un autoclave et équipé d'un agitateur, 5,376 g (24 mmole) du substrat cité aux exemples précédents, 19,2 mg de [(COD)Ru(2-méthylallyl)₂] (0,06 mmole), 0,06 mmole (18,4 mg) de (-)-Me-DuPHOS et 12 ml de CH₂Cl₂. On a ensuite ajouté 0,12 mmole d'éthérate d'HBF₄ (1,2 ml d'une solution 0,1 N dans le dichlorométhane). Après une heure d'agitation, le gobelet contenant un barreau d'agitation a été introduit dans l'autoclave à l'intérieur de la boîte à gants et l'autoclave a été scellé avant d'être pressurisé à l'extérieur. On a purgé les entrées de l'autoclave à l'aide de H₂ et ensuite pressurisé à 2x10⁶ Pa. On a laissé réagir à la température ambiante pendant 17 h. On a dégazé, ouvert l'autoclave et concentré la solution sous pression réduite. On a précipité le catalyseur au pentane, filtré et concentré le filtrat pour obtenir l'Hédione® désirée. Le produit ainsi obtenu (rendement 99%) présentait les rapports isomériques suivants : cis/trans = 97/3 ; (+)-cis/(-)-cis = 80/20.

### Exemple 5

On a procédé de façon similaire à celle décrite dans les exemples 2 à 4, mais en utilisant 2,2 équivalents d'acide tétrafluoroborique (à partir d'une solution 0,2 M d'éthérate d'HBF₄ dans l'éther éthylique) par mode de complexe de Ru(II) dans la préparation du catalyseur, effectuée de façon analogue à celle décrite à l'exemple 1.

Dans les essais résumés aux tableaux ci-après le catalyseur a par ailleurs été employé sous forme solide. Ainsi, on a hydrogéné le 3-oxo-2-pentyl-1-cyclopentène-1-acétate de méthyle (0,5 mmole dans 2 ml de CH₂Cl₂) en présence de la quantité de catalyseur solide indiquée aux tableaux.

Au Tableau II ci-après sont résumés les résultats des réactions d'hydrogénation effectuées avec différents catalyseurs (variation de la nature du ligand), dans les concentrations indiquées, à une pression d'hydrogène d'environ 100 bar (10x10⁶ Pa), température ambiante et pendant une durée de réaction d'environ 22 h.

Le Tableau III résume les résultats obtenus avec différents catalyseurs (variation de la nature du ligand) et en variant plusieurs paramètres de la réaction. Toutes les réactions ont été effectuées à température ambiante, sauf indication contraire.

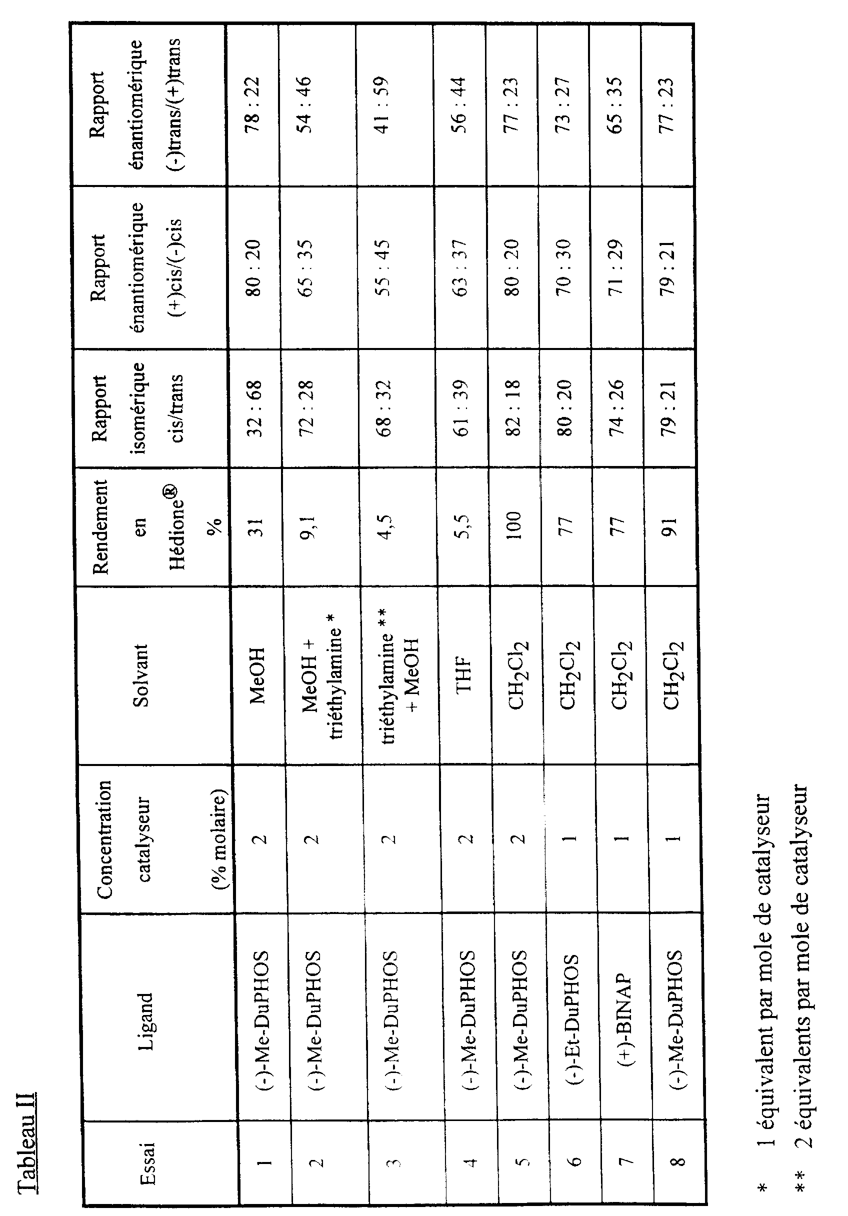

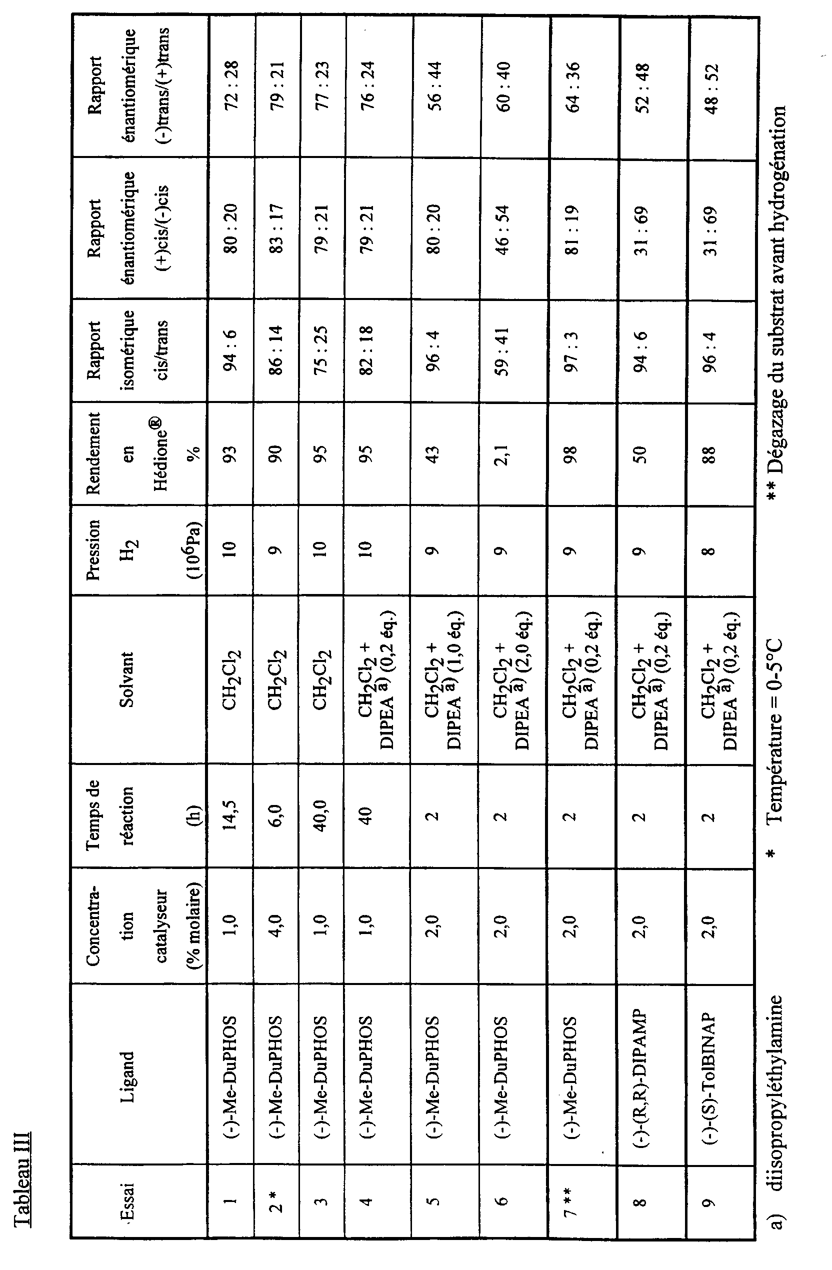

### Exemple 6

Dans une boîte à gants sous argon et à température ambiante on a dissous, dans un gobelet en verre adapté à l'usage dans un autoclave et équipé d'un agitateur, 38 mg de [(COD)Ru(2-méthylallyl)₂] (0,12 mmole), et 36 mg (0,12 mmole) de (-)-Me-DuPHOS dans 5,38 g (24 mmole) de 3-oxo-2-pentyl-1-cyclopentène-acétate de méthyle. On a ensuite ajouté doucement 39 mg d'éthérate d'HBF₄ (0,24 mmole ; 32,7 µl, seringue) en agitant. Après 4 heures d'agitation, on a pris dans un autre gobelet 3,4 ml de cette solution catalytique (contenant environ 0,075 mmole de catalyseur) et on lui a ajouté encore 8,38 g (37,4 mmole) de 3-oxo-2-pentyl-1-cyclopentène-1-acétate de méthyle (quantité totale environ 53 mmole). En opérant comme avant, on a hydrogéné le substrat à une pression de 5x10⁶ Pa de H₂. On a laissé réagir à la température ambiante pendant 20 h. On a dégazé, ouvert l'autoclave et précipité le catalyseur à l'aide de pentane, filtré et concentré le filtrat pour obtenir l'Hédione® désirée. Le produit ainsi obtenu (rendement 99% basé sur le substrat susmentionné) présentait les rapports isomériques suivants : cis/trans = 97/3 : (+)-cis/(-)-cis = 80/20, 60% e.e.

En procédant dans des conditions similaires, mais en variant la nature du solvant d'hydrogénation, c'est-à-dire du mélange contenant le [(COD)Ru(2-méthallyl)₂] et le (-)-Me-DuPHOS, ainsi que celle du solvant de l'éthérate d'acide tétrafluoroborique, on a obtenu les résultats indiqués au Tableau ci-après. Le substrat (II) hydrogéné était toujours le 3-oxo-2-pentyl-1-cyclopentène-1-acétate de méthyle (DHH) et la concentration de catalyseur était d'environ 2% molaire, par rapport audit substrat, sauf indication contraire. Lorsque l'éthérate d'acide tétrafluoroborique est utilisé en solution, sa concentration dans celle-ci est toujours de l'ordre d'environ 0.1 M (moles/l).

On a obtenu systématiquement des rendements de l'ordre de 99% en Hédione®, basé sur le substrat (II) de départ présent dans le milieu d'hydrogénation. Bien entendu, lorsqu'on a utilisé des rapports catalyseur/substrat de l'ordre de 0,5% molaire ou inférieurs, des temps de réaction plus longs (de l'ordre de 6 à 20 h) ont été nécessaires pour obtenir une conversion complète et quantitative du substrat, alors que des durées d'hydrogénation de 1 à 3 heures suffisaient généralement pour obtenir ce résultat lorsque le catalyseur était présent dans des concentrations de l'ordre de 1% molaire ou plus, par rappport au substrat.
On a ainsi constaté à chaque fois une absence presque complète de pertes, avec seulement des traces de résidus (∼ 1%) lorsqu'on a distillé le produit de l'hydrogénation au four à boules ou par distillation Leybold ("thin film distillation").

**Tableau IV**

| Essai | Solvant d'hydrogénation | Solvant du HBF₄.Et₂O | Rapport isomérique cis/trans | Rapport énantiomérique (+)-cis/(-)-cis |
|---|---|---|---|---|
| 1 | dichloroéthane | dichloroéthane | 99 : 1 | 70 : 30 |
| 2 | pivalate de méthyle | dichlorométane | 98,9 : 1,1 | 76 : 24 |
| 3 | acétate d'éthyle | dichlorométhane | 99,3 : 0,7 | 73 : 27 |
| 4 | acétate d'isopropyle | dichlorométhane | 99,3 : 0,7 | 72 : 28 |
| 5 | 2-butanone | dichlorométhane | 98,9 : 1,1 | 79 : 21 |
| 6 | acétate de méthyle | acétate de méthyle | 98,5 : 1,5 | 75 : 25 |
| 7 | acétate de méthyle | acétate de méthyle/dichlorométhane | 99,3 : 0,7 | 74 : 26 |
| 8 | acétate de méthyle | dichlorométhane | 99 : 1 | 77 : 23 |
| 9 | acétate de méthyle/DHH* | acétate de méthyle | 98,5 : 1,5 | 75 : 25 |
| 10 | acétone | dichlorométhane | 99,5 : 0,5 | 79 : 21 |
| 11 | DHH* | acétate de méthyle | 98,2 : 1,8 | 81 : 19 |
| 12 | DHH* | --- | 98,2 : 1,8 | 81 : 19 |
| 13 | 3-pentanone | dichlorométhane | 98,9 : 1,1 | 79 : 21 |
| 14 | tétrahydrofurane | dichlorométhane | pas d'hydrogénation | |
| 15 | toluène | dichlorométhane | pas d'hydrogénation | |
| 16 | N-méthylformamide | dichlorométhane | pas d'hydrogénation | |
| 17 | N,N-diméthylformamide | dichlorométhane | pas d'hydrogénation | |
| 18 | éther sulfurique | dichlorométhane | réaction très lente | |
| 19 | chloroforme | chloroforme | pas d'hydrogénation | |
| 20 | 2-méthylpropanoate de méthyle | dichlorométhane | réaction très lente | |
| 21** | DHH* | --- | 96,7 : 3,3 | 80 : 20 |

| | | | | |
|---|---|---|---|---|
| * 3-oxo-2-pentyl-1-cyclopentène-1-acétate de méthyle | | | | |
| ** concentration catalyseur 0,15 mole % | | | | |

### Exemple 7

On a procédé de façon analogue à celle décrite à l'exemple 6, mais en utilisant 100 g (446 mmole) de 3-oxo-2-pentyl-1-cyclopentène-acétate de méthyle, 427 mg (1,34 mmole) de [(COD)Ru(2-méthylallyl)₂], 410 mg (1,34 mmole) de (-)-Me-DuPHOS et 26,5 ml de HBF₄.O(C₂H₅)₂ dans le dichlorométhane (0,1 M, 2,68 mmole). Ceci correspond à une concentration de 0,3 mole % de catalyseur par rapport au substrat.
On a ajouté 170 ml de dichlorométhane et hydrogéné le mélange, à une température de 7,5°C et une pression de 5x10⁶ Pa pendant 70 h pour obtenir le (+)-(1R)-cis-3-oxo-2-pentyl-1-cyclopentaneacétate de méthyle avec 99% de rendement, rapport cis/trans = 99,2/0,8 et rapport (+)-cis/(-)-cis = 84/16.

### Exemple 8

On a préparé des catalyseurs selon l'invention en utilisant en tant que ligand le (-)-Me-DuPHOS et en variant la nature de l'acide de formule HX.
Les acides de HBF₄, HPF₆, HSbF₆ et HAsF₆ (sous forme d'éthérates) ont été préparés en faisant réagir 0,2 mmole du sel d'argent correspondant, dans 0,8 ml de dichlorométhane, avec 0,2 ml de solution anhydre de HCl 1 M dans l'éther.
Le chlorure d'argent a précipité instantanément et on a obtenu 1 ml de solution d'acide 0,2 M, à savoir, HBF₄, HPF₆, HSbF₆ ou HAsF₆.

Dans un réacteur, on a ensuite introduit 6,4 mg de [(COD)Ru(2-méthylallyl)₂] (0,02 mmole), 6,4 mg de (-)-Me-DuPHOS (0,021 mmole) et 0,224 g de 3-oxo-2-pentyl-1-cyclopentèneacétate de méthyle dans 2 ml de dichlorométhane. La solution ainsi obtenue a été traitée avec 0,20 ou 0,16 ml (2 ou 1,6 équivalents molaires du complexe de ruthénium) de la solution d'acide susmentionnée.

Le milieu a ensuite été hydrogéné à température ambiante et 10x10⁶ Pa pour fournir les résultats résumés au tableau ci-après. Il convient de noter que ces essais n'ont pas été optimisés, ce qui explique les pauvres résultats dans certains cas.

| Essai | Acide | Equivalents d'acide | Temps réaction (h) | Rendement en Hédione® | Rapport cis/trans | Rapport (+)cis/(-)-cis | Rapport (-)trans/(+)-trans |
|---|---|---|---|---|---|---|---|
| 1 | HBF₄ | 2 | 3 | 99,4 | 88 : 12 | 81 : 19 | 79 : 21 |
| 2 | HBF₄ | 1,6 | 3 | 98,7 | 87 : 13 | 82 : 18 | 81 : 19 |
| 3 | HAsF₆ | 2 | 3,5 | 78,7 | 56 : 44 | 79 : 21 | 78 : 22 |
| 4 | HAsF₆ | 1,6 | 3,5 | 99,3 | 82 : 18 | 78 : 22 | 78 : 22 |
| 5 | HPF₆ | 2 | 2 | 2,9 | 51 : 49 | 57 : 43 | 60 : 40 |
| 6 | HPF₆ | 1,6 | 2 | 80,8 | 86 : 14 | 71 : 29 | 69 : 31 |
| 7 | HSbF₆ | 2 | 3 | 98,3 | 48 : 52 | 75 : 25 | 74 : 26 |
| 8 | HSbF₆ | 1,6 | 3 | 99,0 | 84 : 16 | 75 : 25 | 74 : 26 |

Des résultats similaires ont été obtenus lorsqu'on a ajouté au milieu d'hydrogénation de la diisopropyléthylamine [0,040 ml de solution 0,1 M ; 20% molaire par rapport au Ru(II)]. On a alors observé une amélioration sensible (de l'ordre de 10%) du rapport isomérique cis/trans.

### Exemple 9

En procédant de façon similaire à celle décrite aux exemples précédents, on a testé l'activité de différents catalyseurs racémiques ou achiraux selon l'invention, préparés comme il est décrit à l'exemple 1, en utilisant HBF₄ comme acide (sous forme de son éthérate) et en variant la nature du ligand; dans l'hydrogénation du 3-oxo-2-pentyl-1-cyclopentène-acétate de méthyle.

Toutes les hydrogénations ont eu lieu dans le dichlorométhane et à température ambiante. Les résultats sont indiqués au Tableau. Il convient de noter que ces essais n'ont pas été optimisés, ce qui explique les pauvres taux de conversion du substrat dans certains cas.

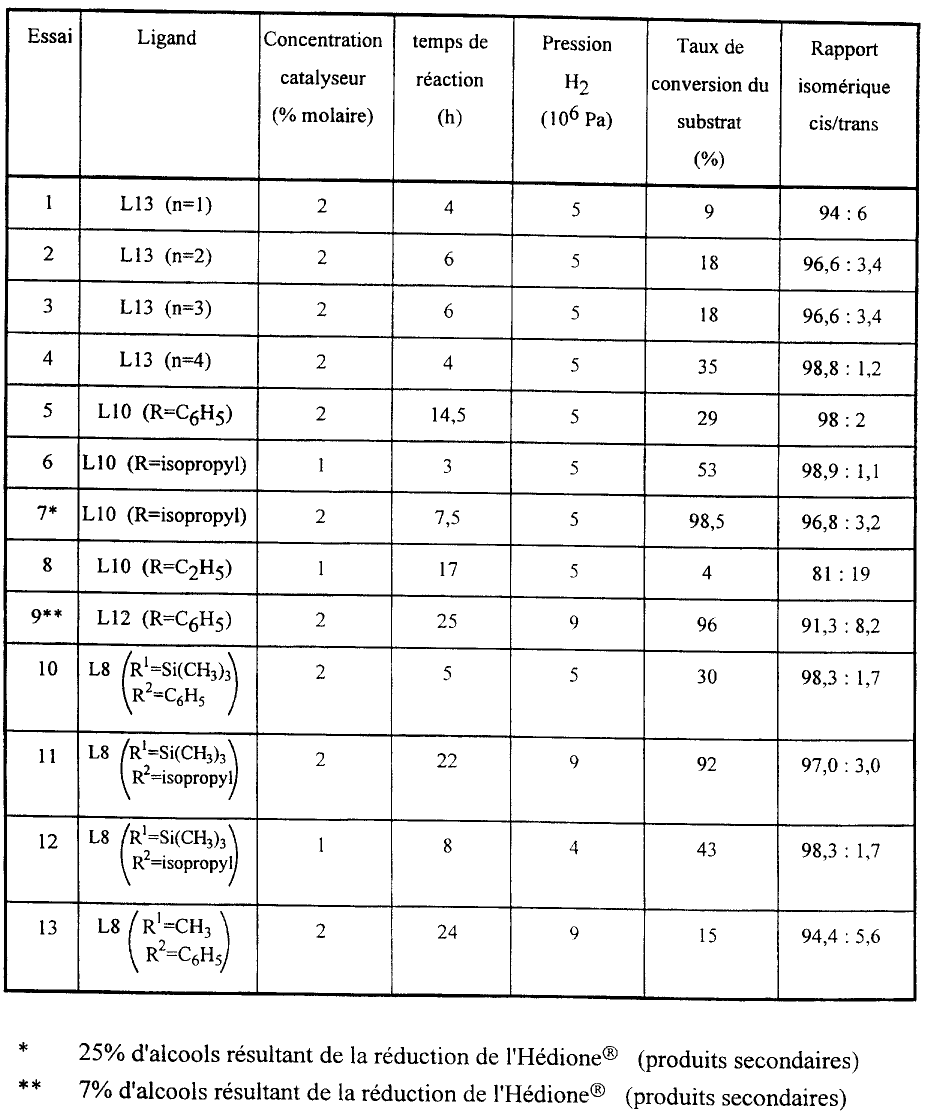

### Exemple 10

A. Dans une boîte à gants sous Ar, on a introduit dans un gobelet en verre de 250 ml 4,05 g de HBF₄.Et₂O (Et=C₂H₅ ; 25,0 mmole, 3,40 ml) à l'aide d'une pipette et ajouté environ 100 ml de dichlorométhane. On a ensuite agité pour dissoudre l'éthérate et rempli le gobelet pour parfaire le volume à 250 ml. La solution 0,1 M (moles/l) de HBF₄.Et₂O ainsi obtenue a été utilisée telle quelle dans la préparation du catalyseur.
B. Dans la boîte à gants, on a mélangé dans un récipient en verte 16,0 mg (0,050 mmole) de [Ru(COD)(2-methallyl)₂] et 15,0 mg (0,050 mmole) de (-)-Me-DuPHOS et dissous dans 4 ml de CH₂Cl₂. On a ensuite ajouté lentement, via une seringue (environ 1 min), 1 ml (0,1 mmole) de la solution d'éthérate de HBF₄ préparée en A, à température ambiante. La solution rouge-brun résultante, à 0,01 M (moles/l) dans le catalyseur, a été laissée au repos pendant 2 h avant utilisation. Elle reste active pendant plus d'une semaine, lorsque stockée à température ambiante dans la boîte à gants.
C. A l'intérieur de la boîte à gants, dans un gobelet en verte pouvant être transféré dans un autoclave, on a ajouté 224 mg (1 mmole) de 3-oxo-2-pentyl-1-cyclopentène-1-acétate de méthyle à 2 ml de la solution catalytique obtenue sous B (0,02 mmole, 2 mole % par rapport au substrat). La solution ainsi obtenue a ensuite été transférée dans une autoclave et agitée pendant 2 h, à 90 bar (9x10⁶ Pa) d'hydrogène, à température ambiante. L'autoclave a été presque entièrement dégazée et ouverte à l'intérieur de la boîte à gants. Le mélange de réaction jaune-orange a été évaporé sous vide pour enlever le dichlorométhane. Le résidu solide et le liquide jauneorange ainsi obtenus ont été traités au pentane et la suspension filtrée à travers un filtre en Teflon® de 0,22µ pour précipiter le catalyseur. Le solide qui reste est lavé au pentane et filtré à la fois à travers le même filtre. Les solutions combinées dans le pentane contenaient l'Hédione® désirée avec un rapport cis/trans=98,5/1,5 et un rapport (+)-cis/(-)-cis=79/21.
D. Un deuxième cycle d'hydrogénation a été effectué avec le catalyseur resté dans le filtre Teflon®. Ce dernier a été lavé avec 2 ml de CH₂Cl₂ et ce dichlorométhane utilisé pour dissoudre le précipitat resté dans le récipient. On a ajouté 224 mg du substrat cité en C à cette solution et ensuite hydrogéné comme décrit en C pour obtenir à nouveau l'Hédione® avec un rapport cis/trans de 98,8/1,2 et un rapport (+)-cis/(-)-cis de 79/21.
B'. Selon un mode d'exécution alternatif à celui décrit sous B, on a également préparé le catalyseur en procédant ainsi : on a d'abord ajouté la solution de HBF₄.Et₂O dans le dichlorométhane décrite en A au (-)-Me-DuPHOS et évaporé l'éther et le dichlorométhane. Le solide constitué par le sel onium du ligand ainsi obtenu a ensuite été redissous dans du dichlorométhane pur et ajouté au [Ru(COD)(2-methallyl)₂]. La réaction a lieu avec libération d'isobutène. Le catalyseur ainsi obtenu a été utilisé dans des conditions identiques à celles décrites sous C pour fournir la (+)-cis-Hédione® avec les mêmes caractéristiques.
C' Des résultats similaires à ceux décrits sous C ont été obtenus losqu'on a utilisé dans la préparation du catalyseur et à la place du complexe [Ru(COD)(2-methallyl)₂], le bis(2,4-diméthylpentadiényl)ruthénium (voir par exemple, L. Stahl et al., Organometallics, 1983, 2, 1229) ou le bis(2,4-diméthyl-1-oxapentadiényl)ruthénium (voir T. Schmidt et al., J. Chem. Soc. Chem. Comm., 1991, 1427).
C''. Par ailleurs, l'utilisation dans l'hydrogénation décrite sous C d'un catalyseur préparé de façon identique à celle décrite sous B, mais en employant comme ligand le (-)-JOSIPHOS (ligand L11, R=cyclohexyl, R'=phényle; origine : STREM Chemicals Inc.), a permis d'obtenir l'Hédione® avec un rapport cis/trans de 98/2 et un rapport (+)-cis/(-)-cis de 77/23.

### Exemple 11

On a procédé de façon similaire à celle décrite à l'Exemple 10A à C, mais en utilisant 4,171 g (13,056 mmole) de [Ru(COD)(2-méthallyl)₂], 4,000 g (13,056 mmole) de (-)-Me DuPHOS, 1000 g (4,458 mole) de 3-oxo-2-pentyl-1-cyclopentène-1-acétate de méthyle, 261 ml (26,10 mmole) de solution 0,1 M de HBF₄.Et₂O dans le dichlorométhane et 1,71 de CH₂Cl₂.
La réaction d'hydrogénation a été effectuée à 7,5° et sous une pression d'hydrogène de 35 bar (3,5x10⁶ Pa), pendant 24 h, sous agitation (1000-2000 rpm).
Après évaporation du solvant, on a obtenu 1095 g de produit brun qui a été traité au pentane et la solution filtrée comme décrit précédemment. Les extraits de pentane combinés ont été concentrés sous vide pour fournir 978 g de produit, dont la distillation en couche mince (appareil de type Leybold, 86°/0,1 mbar) a fourni 932 g de produit incolore consistant en l'Hédione® désirée ayant un rapport cis/trans de 98/2 et (+)-cis/(-)-cis de 85/15.

## Revendications

1. Catalyseur de ruthénium (II) comprenant des ligands constitués par des phosphines bidentées, caractérisé en ce qu'il est susceptible d'être obtenu par un procédé qui comprend le traitement d'un complexe de Ru(II) approprié et d'un ligand diphosphiné bidenté, présents en quantités équimolaires, avec un acide de formule HX, où X représente un anion non coordinant, cet acide étant utilisé dans une proportion ne dépassant pas 2 équivalents molaires par mole du complexe de Ru(II), le traitement ayant lieu dans un milieu non complexant ou faiblement complexant et sous atmosphère inerte.

2. Catalyseur selon la revendication 1, caractérisé en ce que le complexe de Ru(II) est sélectionné dans le groupe des composés de Ru(II) de type [(diène)Ru(allyl)₂] ou [bis(pentadiényl)Ru].

3. Catalyseur selon la revendication 2, caractérisé en ce que le complexe de ruthénium est le [(COD)Ru(2-méthallyl)₂], le [bis-(2,4-diméthylpentadiényl)Ru] ou le [bis-(2,4-diméthyl-1-oxapentadiényl)Ru].

4. Catalyseur selon l'une des revendications 1 à 3, caractérisé en ce que le ligand diphosphiné est sélectionné dans le groupe constitué par les ligands chiraux connus sous les abréviations de Me-DuPHOS, Et-DuPHOS, BINAP, TolBINAP, SKEWPHOS et JOSIPHOS.

5. Catalyseur selon la revendication 4 caractérisé en ce que le ligand phosphiné bidenté est sélectionnée dans le groupe des diphosphines chirales connues sous les abréviations de Me-DuPHOS, SKEWPHOS et JOSIPHOS.

6. Catalyseur selon la revendication 5, caractérisé en ce que le ligand est le (R,R)-(-)-Me-DuPHOS.

7. Catalyseur selon l'une des revendications précédentes, caractérisé en ce que l'acide est sélectionné dans le groupe constitué par HBF₄, HPF₆, HSbF₆, HAsF₆, et HB[3,5-(CF₃)₂C₆H₄]₄.

8. Catalyseur selon la revendication 7, caractérisé en ce qu'on utilise le HBF₄, sous forme de son éthérate.

9. Catalyseur selon la revendication 7 ou 8, caractérisé en ce que l'acide est utilisé dans une proportion comprise entre 1,5 et 2 équivalents molaires, par mole de complexe de Ru(II).

10. Catalyseur selon l'une des revendications 1 à 9, caractérisé en ce que le traitement a lieu en présence d'un solvant organique non complexant ou faiblement complexant et/ou d'un substrat de formule 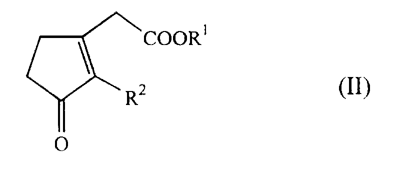 dans laquelle R¹ représente un radical alkyle linéaire ou ramifié de C₁ à C₄ et R² représente un reste d'hydrocarbure saturé ou insaturé, linéaire ou ramifié, de C₁ à C₈.

11. Catalyseur selon la revendication 10, caractérisé en ce que le traitement a lieu uniquement en présence d'un substrat de formule (II) telle que définie à la revendication 10.

12. Catalyseur selon la revendication 10, caractérisé en ce que le solvant est choisi dans le groupe constitué par le dichlorométhane, le dichloroéthane, le pivalate de méthyle, l'acétate de méthyle, l'acétate d'éthyle, l'acétate d'isopropyle, l'acétone, la 2-butanone, la 3-pentanone et tout mélange de deux ou plusieurs de ces solvants.

13. Catalyseur selon la revendication 12, caractérisé en ce que le solvant est, ou comprend, le dichlorométhane.

14. Catalyseur selon la revendication 10 ou 11, caractérisé en ce que le substrat de formule (II) est le 3-oxo-2-pentyl-1-cyclopentène-1-acétate de méthyle.

15. Catalyseur selon la revendication 1, caractérisé en ce que le traitement a lieu à température ambiante.

16. Procédé pour la préparation d'un catalyseur de Ru(II) selon la revendication 1, caractérisé en ce qu'on fait réagir, sous atmosphère inerte et dans un milieu non complexant ou faiblement complexant, des quantités équimolaires d'un complexe de Ru(II) approprié et d'un ligand diphosphiné bidenté, avec un acide de formule HX, où X représente un anion non coordinant, cet acide étant utilisé dans une proportion ne dépassant pas 2 équivalents molaires par mole de complexe de Ru(II).

17. Procédé selon la revendication 16, caractérisé en ce que la réaction a lieu en présence d'un solvant organique non complexant ou faiblement complexant et/ou d'un substrat de formule 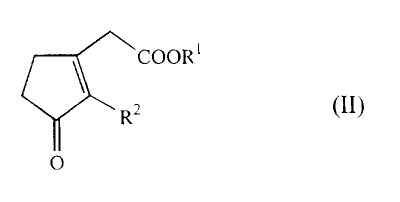 dans laquelle R¹ représente un radical alkyle linéaire ou ramifié de C₁ à C₄ et R² représente un reste d'hydrocarbure saturé ou insaturé, linéaire ou ramifié, de C₁ à C₈.

18. Utilisation en tant que catalyseur d'hydrogénation du catalyseur de ruthénium (II) faisant l'objet de l'une des revendications 1 à 15.

19. Procédé pour la préparation d'un composé de formule 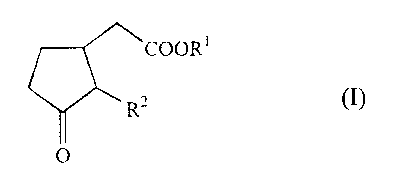 dans laquelle R¹ représente un radical alkyle linéaire ou ramifié de C₁ à C₄ et R² représente un reste d'hydrocarbure saturé ou insaturé, linéaire ou ramifié de C₁ à C₈, essentiellement sous forme d'un isomère de configuration cyclanique cis, caractérisé en ce qu'on soumet à une hydrogénation catalytique un substrat de formule 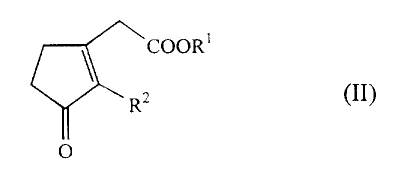 dans laquelle R¹ et R² ont le sens indiqué ci-dessus, en présence d'un catalyseur de Ru(II) selon l'une des revendications 1 à 15, et à une pression d'hydrogène comprise entre 10 et 100 bar.

20. Procédé selon la revendication 19, caractérisé en ce qu'on utilise un catalyseur comprenant en tant que ligand une diphosphine chirale appropriée, pour obtenir le composé (I) essentiellement sous forme d'un isomère optiquement actif de configuration cyclanique (1R)-cis.

21. Procédé selon la revendication 19 ou 20, caractérisé en ce que l'hydrogénation catalytique a lieu dans un solvant non-complexant ou faiblement complexant dans les conditions de la réaction.

22. Procédé selon l'une des revendications 19 à 21, caractérisé en ce que le catalyseur de Ru(II) est engendre in situ, éventuellement en présence du substrat de formule (II).

23. Procédé selon la revendication 21, caractérisé en ce que le solvant non-complexant est choisi dans le groupe défini à la revendication 12.

24. Procédé selon l'une des revendications 19 à 23, caractérisé en ce que le substrat de formule (II) est le 3-oxo-2-pentyl-1-cyclopentène-1-acétate de méthyle et en ce qu'on obtient essentiellement le (+)-(1R)-cis-3-oxo-2-pentyl-1-cyclopentaneacétate de méthyle.

25. Procédé selon la revendication 24, caractérisé en ce que l'hydrogénation a lieu en présence d'un catalyseur selon la revendication 5 ou 6.

26. Procédé selon l'une des revendications 19 à 25, caractérisé en ce que le catalyseur est présent dans une concentration comprise entre 0,1 et 2 mole%, par rapport au substrat.

## Claims

1. Ruthenium (II) catalyst comprising ligands formed of bidentate phosphines, characterized in that it is obtainable by a process which comprises treating an appropriate Ru(II) complex and a bidentate diphosphine ligand, present in equimolar amounts, with an acid of formula HX, wherein X is a non-coordinating anion, said acid being used in a ratio which does not exceed 2 molar equivalents per mole of the Ru(II) complex, the treatment being carried out in a non-coordinating or weakly coordinating medium and under an inert atmosphere.

2. Catalyst according to claim 1, characterized in that the Ru(II) complex is selected from the group of Ru(II) compounds of the type [(diene)Ru(allyl)₂] or [bis(pentadienyl)Ru].

3. Catalyst according to claim 2, characterized in that the ruthenium complex is [(COD)Ru(2-methallyl)₂], [bis-(2,4-dimethylpentadienyl)Ru] or [bis(2,4-dimethyl-1-oxapentadienyl)Ru].

4. Catalyst according to any one of claims 1 to 3, characterized in that the diphosphine ligand is selected from the group consisting of the chiral ligands known under the abbreviations of Me-DuPHOS, Et-DuPHOS, BINAP, TolBINAP, SKEWPHOS and JOSIPHOS.

5. Catalyst according to claim 4, characterized in that the bidentate phosphine ligand is selected from the group consisting of the chiral diphosphines known under the abbreviations of Me-DuPHOS, SKEWPHOS and JOSIPHOS.

6. Catalyst according to claim 5, characterized in that the ligand is (R,R)-(-)-Me-DuPHOS.

7. Catalyst according to any one of the previous claims, characterized in that the acid is selected from the group consisting of HBF₄, HPF₆, HSbF₆, HAsF₆, and HB[3,5-(CF₃)₂C₆H₄]₄.

8. Catalyst according to claim 7, characterized in that HBF₄ is used in the form of its etherate.

9. Catalyst according to claim 7 or 8, characterized in that the acid is used in a proportion of 1.5 to 2 molar equivalents per mole of Ru(II) complex.

10. Catalyst according to any of claims 1 to 9, characterized in that the treatment is carried out in the presence of a non-coordinating or weakly coordinating organic solvent and/or of a substrate of formula 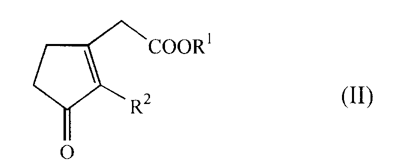 wherein R¹ represents a linear or branched alkyl radical from C₁ to C₄ and R² represents a saturated or unsaturated, linear or branched hydrocarbon rest from C₁ to C₈.

11. Catalyst according to claim 10, characterized in that the treatment is carried out only in the presence of a substrate of formula (II) as defined in claim 10.

12. Catalyst according to claim 10, characterized in that the solvent is selected from the group consisting of dichloromethane, dichloroethane, methyl pivalate, methyl acetate, ethyl acetate, isopropyl acetate, acetone, 2-butanone, 3-pentanone and any mixture of two or several of said solvents.

13. Catalyst according to claim 12, characterized in that the solvent is or comprises dichloromethane.

14. Catalyst according to claim 10 or 11, characterized in that the substrate of formula (II) is methyl 3-oxo-2-pentyl-1-cyclopentene-1-acetate.

15. Catalyst according to claim 1, characterized in that the treatment is carried out at room temperature.

16. Process for the preparation of a catalyst of Ru(II) according to claim 1, characterized in that equimolar amounts of an appropriate Ru(II) complex and of a bidentate diphosphine ligand are reacted, under an inert atmosphere and in a non-coordinating or weakly coordinating medium, with an acid of formula HX, wherein X represents a non-coordinating anion, said acid being used in a proportion not exceeding 2 molar equivalents per mole of Ru(II) complex.

17. Process according to claim 16, characterized in that the reaction is carried out in the presence of an organic non-coordinating or weakly coordinating solvent and/or a substrate of formula 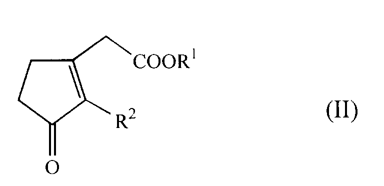 wherein R¹ is a linear or branched alkyl radical from C₁ to C₄ and R² is a saturated or unsaturated, linear or branched hydrocarbon rest from C₁ to C₈.

18. Use of a Ru(II) catalyst according to any of claims 1 to 15 as a hydrogenation catalyst.

19. Process for the preparation of a compound of formula 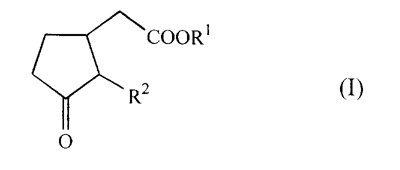 wherein R¹ represents a linear or branched alkyl radical from C₁ to C₄ and R² represents a saturated or unsaturated, linear or branched hydrocarbon rest from C₁ to C₈, essentially in the form of the cis-configuration isomer, characterized in that a substrate of formula 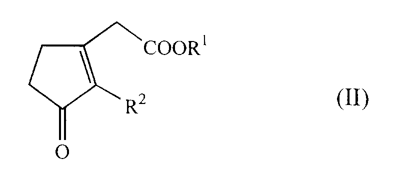 in which R¹ and R² have the meaning indicated above, is hydrogenated in the presence of a Ru(II) catalyst according to any of claims 1 to 15, at a hydrogen pressure from 10 to 100 bar.

20. Process according to claim 19, characterized in that there is used a catalyst comprising as a ligand an appropriate chiral diphosphine, to obtain compound (I) essentially in the form of an optically active isomer of (1R)-cis configuration.

21. Process according to claim 19 or 20, characterized in that the catalytic hydrogenation is carried out in a non-coordinating or weakly coordinating solvent under the reaction conditions.

22. Process according to any of claims 19 to 21, characterized in that the Ru(II) catalyst is formed in situ, optionally in the presence of the substrate of formula (II).

23. Process according to claim 21, characterized in that the non-coordinating solvent is selected from the group defined in claim 12.

24. Process according to any of claims 19 to 23, characterized in that the substrate of formula (II) is methyl 3-oxo-2-pentyl-1-cyclopentene-1-acetate and in that essentially methyl (+)-(1R)-cis-3-oxo-2-pentyl-1-cyclopentaneacetate is obtained.

25. Process according to claim 24, characterized in that the hydrogenation is carried out in the presence of a catalyst according to claim 5 or 6.

26. Process according to any of claims 19 to 25, characterized in that the catalyst is present in a concentration from 0.1 to 2 mole%, with respect to the substrate.

## Patentansprüche

1. Ruthenium (II)-Katalysator mit Liganden, welche aus zweizähnigen Phosphinen bestehen, dadurch gekennzeichnet, daß er durch ein Verfahren herstellbar ist, welches die Behandlung eines geeigneten Ru(II)-Komplexes und eines zweizähnigen Diphosphinliganden, die in äquimolaren Mengen vorliegen, mit einer Säure mit der Formel HX umfaßt, wobei X für ein nicht-koordinierendes Anion steht, diese Säure in einem Verhältnis eingesetzt wird, das 2 Moläquivalente pro Mol des Ru(II)-Komplexes nicht übersteigt, und die Behandlung in einem nicht bzw. nur schwach komplexierenden Medium und unter Inertatmosphäre stattfindet.

2. Katalysator nach Anspruch 1, dadurch gekennzeichnet, daß der Ru(II)-Komplex aus der Gruppe der Ru(II)-Verbindungen vom Typ [(Dien)Ru(allyl)₂] oder [Bis(pentadienyl)Ru] ausgewählt ist.

3. Katalysator nach Anspruch 2, dadurch gekennzeichnet, daß der Rutheniumkomplex [(COD)Ru(2-methallyl)₂], [Bis-(2,4-dimethylpentadienyl)Ru] oder [Bis-(2,4-dimethyl-1-oxapentadienyl)Ru] ist.

4. Katalysator nach einem der Ansprüche 1 bis 3, dadurch gekennzeichnet, daß der Diphosphinligand aus der Gruppe ausgewählt ist, welche aus den unter den Abkürzungen Me-DuPHOS, Et-DuPHOS, BINAP, TolBINAP, SKEWPHOS und JOSIPHOS bekannten chiralen Liganden besteht.

5. Katalysator nach Anspruch 4, dadurch gekennzeichnet, daß der zweizähnige Diphosphinligand aus der Gruppe von chiralen Diphosphinen ausgewählt ist, welche unter den Abkürzungen Me-DuPHOS, SKEWPHOS und JOSIPHOS bekannt sind.

6. Katalysator nach Anspruch 5, dadurch gekennzeichnet, daß der Ligand (R,R)-(-)-Me-DuPHOS ist.

7. Katalysator nach einem der vorhergehenden Ansprüche, dadurch gekennzeichnet, daß die Säure aus der Gruppe ausgewählt ist, welche aus HBF₄, HPF₆, HSbF₆, HAsF₆ und HB[3,5-(CF₃)₂C₆H₄]₄ besteht.

8. Katalysator nach Anspruch 7, dadurch gekennzeichnet, daß HBF₄ in Form seines Etherates eingesetzt wird.

9. Katalysator nach Anspruch 7 oder 8, dadurch gekennzeichnet, daß die Säure in einem Verhältnis eingesetzt wird, das zwischen 1,5 und 2 Moläquivalenten pro Mol Ru(II)-Komplex liegt.

10. Katalysator nach einem der Ansprüche 1 bis 9, dadurch gekennzeichnet, daß die Behandlung in Gegenwart eines nicht bzw. nur schwach komplexierenden Lösungsmittels und/oder eines Substrates mit der Formel 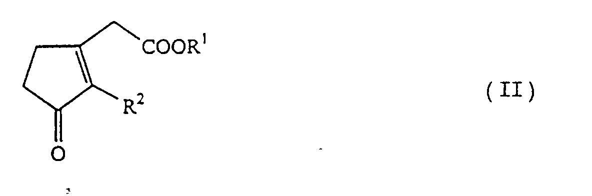 stattfindet, wobei R¹ für eine lineare oder verzweigte C₁-C₄-Alkylgruppe steht und R² für einen gesättigten oder ungesättigten, linearen oder verzweigten C₁-C₈-Kohlenwasserstoffrest steht.

11. Katalysator nach Anspruch 10, dadurch gekennzeichnet, daß die Behandlung nur in Gegenwart eines Substrates mit der Formel (II) gemäß der Definition von Anspruch 10 stattfindet.

12. Katalysator nach Anspruch 10, dadurch gekennzeichnet, daß das Lösungsmittel aus der Gruppe ausgewählt ist, welche aus Dichlormethan, Dichlorethan, Methylpivalat, Methylacetat, Ethylacetat, Isopropylacetat, Aceton, 2-Butanon, 3-Pentanon und jeglicher Mischung von zweien oder mehr dieser Lösungsmittel besteht.

13. Katalysator nach Anspruch 12, dadurch gekennzeichnet, daß das Lösungsmittel Dichlormethan ist bzw. aufweist.

14. Katalysator nach Anspruch 10 oder 11, dadurch gekennzeichnet, daß das Substrat mit der Formel (II) Methyl 3-Oxo-2-pentyl-1-cyclopenten-1-acetat ist.

15. Katalysator nach Anspruch 1, dadurch gekennzeichnet, daß die Behandlung bei Umgebungstemperatur stattfindet.

16. Verfahren zur Herstellung eines Ru(II)-Katalysators gemäß Anspruch 1, dadurch gekennzeichnet, daß unter Inertatmosphäre und in einem nicht bzw. nur schwach komplexierenden Medium äquimolare Mengen eines geeigneten Ru(II)-Komplexes und eines zweizähnigen Diphosphinliganden mit einer Säure mit der Formel HX umgesetzt werden, wobei X für ein nicht-koordinierendes Anion steht und diese Säure in einem Verhältnis eingesetzt wird, das 2 Moläquivalente pro Mol Ru(II)-Komplex nicht übersteigt.

17. Verfahren nach Anspruch 16, dadurch gekennzeichnet, daß die Reaktion in Gegenwart eines nicht bzw. nur schwach komplexierenden organischen Lösungsmittels und/oder eines Substrates mit der Formel 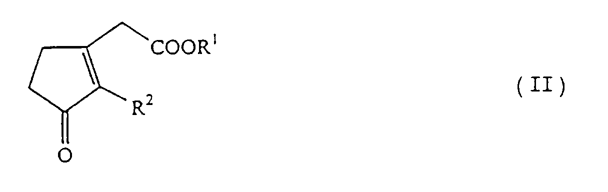 stattfindet, wobei R¹ für eine lineare oder verzweigte C₁-C₄-Alkylgruppe und R² für einen gesättigten oder ungesättigten, linearen oder verzweigten C₁-C₈-Kohlenwasserstoffrest steht.

18. Verwendung des den Gegenstand eines der Ansprüche 1 bis 15 darstellenden Ruthenium (II)-Katalysators als Hydrierungskatalysator.

19. Verfahren zur Herstellung einer Verbindung mit der Formel 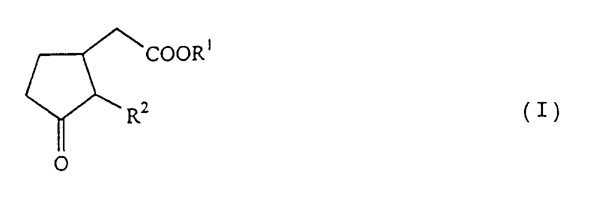 wobei R¹ für eine lineare oder verzweigte C₁-C₄-Alkylgruppe und R² für einen gesättigten oder ungesättigten, linearen oder verzweigten C₁-C₈-Kohlenwasserstoffrest steht, im wesentlichen in Form eines Isomers mit cis-Cyclankonfiguration, dadurch gekennzeichnet, daß ein Substrat mit der Formel 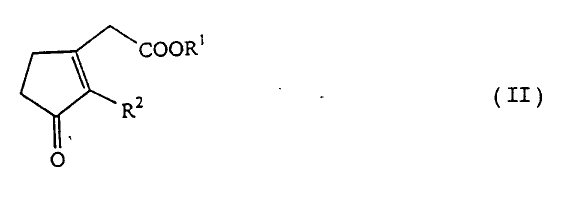 wobei R¹ und R² die obenstehende Bedeutung haben, einer katalytischen Hydrierung in Gegenwart eines Ru(II)-Katalysators nach einem der Ansprüche 1 bis 15 und bei einem zwischen 10 und 100 bar liegenden Wasserstoffdruck unterzogen wird.

20. Verfahren nach Anspruch 19, dadurch gekennzeichnet, daß ein Katalysator verwendet wird, welcher als Ligand ein geeignetes chirales Diphosphin aufweist, um die Verbindung (I) im wesentlichen in Form eines optisch aktiven Isomers mit (1R)-cis-Cyclankonfiguration herzustellen.

21. Verfahren nach Anspruch 19 oder 20, dadurch gekennzeichnet, daß die katalytische Hydrierung in einem unter den Reaktionsbedingungen nicht bzw. nur schwach komplexierenden Lösungsmittel stattfindet.

22. Verfahren nach einem der Ansprüche 19 bis 21, dadurch gekennzeichnet, daß der Ru(II)-Katalysator in situ erzeugt wird, gegebenenfalls in Gegenwart des Substrates mit der Formel (II).

23. Verfahren nach Anspruch 21, dadurch gekennzeichnet, daß das nicht-komplexierende Lösungsmittel aus der in Anspruch 12 definierten Gruppe ausgewählt ist.

24. Verfahren nach einem der Ansprüche 19 bis 23, dadurch gekennzeichnet, daß das Substrat mit der Formel (II) Methyl 3-Oxo-2-pentyl-1-cyclopenten-1-acetat ist, und daS im wesentlichen (+)-Methyl (1R)-cis-3-Oxo-2-pentyl-1-cyclopentanacetat hergestellt wird.

25. Verfahren nach Anspruch 24, dadurch gekennzeichnet, daß die Hydrierung in Gegenwart eines Katalysators gemäß Anspruch 5 oder 6 stattfindet.

26. Verfahren nach einem der Ansprüche 19 bis 25, dadurch gekennzeichnet, daß der Katalysator in einer Konzentration zwischen 0,1 und 2 Mol-% in bezug auf das Substrat vorliegt.
